# EUROPÄISCHE PATENTSCHRIFT

(11) **EP 2 683 680 B1**
(45) Veröffentlichungstag und Bekanntmachung des Hinweises auf die Patenterteilung: **17.06.2015**
(21) Anmeldenummer: 12706272.7
(22) Anmeldetag: 01.03.2012
(51) Int. Cl.: C07C 213/02, C07D 207/04, C07D 207/20, C07D 295/027

(54) **VERFAHREN ZUR HERSTELLUNG VON PRIMÄREN AMINEN DURCH HOMOGEN-KATALYSIERTE ALKOHOLAMINIERUNG**
METHOD FOR PRODUCING PRIMARY AMINES BY MEANS OF HOMOGENEOUSLY-CATALYSED ALCOHOL AMINATION
PROCÉDÉ DE PRODUCTION D'AMINES PRIMAIRES PAR AMINATION DES ALCOOLS EN CATALYSE HOMOGÈNE

(30) Priorität: 08.03.2011 EP 11157335
(43) Veröffentlichungstag der Anmeldung: 15.01.2014
(73) Patentinhaber: BASF SE, 67056 Ludwigshafen (DE)
(72) Erfinder: SCHAUB, Thomas, 67433 Neustadt (DE); BUSCHHAUS, Boris, 69117 Heidelberg (DE); BRINKS, Marion, Kristina, 68165 Mannheim (DE); SCHELWIES, Mathias, 69115 Heidelberg (DE); PACIELLO, Rocco, 67098 Bad Dürkheim (DE); MELDER, Johann-Peter, 67459 Böhl-Iggelheim (DE); MERGER, Martin, 67227 Frankenthal (DE)
(86) Internationale Anmeldenummer: PCT/EP2012/053585
(87) Internationale Veröffentlichungsnummer: WO 2012/119930

(56) Entgegenhaltungen:
- WO-A1-2010/018570
- SEBASTIAN IMM ET AL: "An Efficient and General Synthesis of Primary Amines by Ruthenium-Catalyzed Amination of Secondary Alcohols with Ammonia", ANGEWANDTE CHEMIE. INTERNATIONAL EDITION, WILEY VCH VERLAG, WEINHEIM, Bd. 49, Nr. 44, 25. Oktober 2010 (2010-10-25), Seiten 8126-8129, XP002669003, ISSN: 1433-7851, DOI: 10.1002/ANIE.201002576 [gefunden am 2010-08-02]

## Beschreibung

Die vorliegende Erfindung betrifft ein Verfahren zur Herstellung von primären Aminen durch homogen-katalysierte Alkoholaminierung von Alkoholen und Alkanolaminen mit Ammoniak unter Wasserabspaltung in Gegenwart eines Komplexkatalysators, der mindestens ein Element ausgewählt aus den Gruppen 8, 9 und 10 des Periodensystems enthält und mindestens eines unpolaren Lösungsmittels.

Primäre Amine sind Verbindungen, die mindestens eine primäre Aminogruppe (-NH₂) aufweisen. Primäre Diamine weisen zwei primäre Aminogruppen auf. Primäre Triamine weisen drei primäre Aminogruppen auf. Primäre Polyamine weisen mehr als drei primäre Aminogruppe auf.

Primäre Amine sind wertvolle Produkte mit einer Vielzahl unterschiedlicher Verwendungen, beispielsweise als Lösungsmittel, Stabilisatoren, zur Synthese von Chelat-Bildnern, als Edukte zur Herstellung von Kunstharzen, Inhibitoren, grenzflächenaktiven Substanzen, Zwischenprodukte bei der Herstellung von Kraftstoffadditiven (US 3,275,554 A, DE 2125039 A und DE 36 11 230 A), Tensiden, Arznei- und Planzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Primäre Di-, und Triamine sind wertvolle Produkte mit einer Vielzahl unterschiedlicher Verwendungen, beispielsweise als Lösungsmittel, Stabilisatoren, zur Synthese von Chelat-Bildnern, als Edukte zur Herstellung von Kunstharzen, Arzneimitteln, Inhibitoren, Korrosionsschutzmitteln, Polyurethanen, als Härter für Epoxyharze, und grenzflächenaktiven Substanzen.

Primäre Di- und Triamine werden gegenwärtig durch heterogen-katalysierte Alkoholaminierung von primären Di- und Triolen mit Ammoniak hergestellt. Die WO 2008/006752 A1 beschreibt ein Verfahren zur Herstellung von Aminen durch Umsetzung von primären oder sekundären Alkoholen mit Ammoniak in Gegenwart eines heterogenen Katalysators, der Zirkoniumdioxid und Nickel enthält. Die WO 03/051508 A1 betrifft ein Verfahren zur Aminierung von Alkoholen unter Verwendung von spezifischen heterogenen Cu/Ni/Zr/Sn-Katalysatoren. Aus der EP 0 696 572 A1 sind Nickel-, Kupfer-, Zirkonium- und Molybdänoxid enthaltende heterogene Katalysatoren zur Aminierung von Alkoholen mit Ammoniak und Wasserstoff bekannt. Die Reaktionen werden gemäß den vorstehend genannten Dokumenten bei Temperaturen im Bereich von 150 bis 210 °C und Ammoniakdrücken im Bereich von 30 bis 200 bar durchgeführt. Bei den in den vorstehenden Dokumenten beschriebenen heterogen-katalysierten Verfahren entstehen als Hauptprodukte jedoch die oft unerwünschten Monoaminierungsprodukte und zyklische Amine wie Piperazine, Pyrrolidine und Morpholine. Die gewünschten primären Diamine werden bei den vorstehend beschriebenen Verfahren nicht oder lediglich in äußerst geringen Ausbeuten erhalten. In den vorstehend genannten Dokumenten ist insbesondere die Umsetzung von Diethylenglykol mit Ammoniak beschrieben.

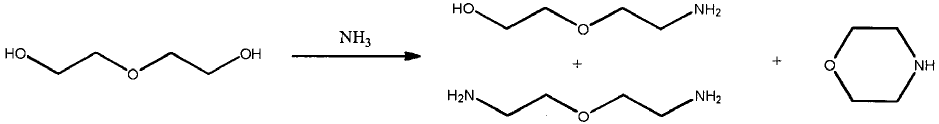

Hierbei werden als Hauptprodukte Monoaminodiethylenglykol und Morpholin erhalten. Das gewünschte zweifach aminierte Diaminodiethylenglykol wird bei den Aminierungsreaktionen der vorstehend genannten Dokumente nicht oder nur in äußerst geringen Ausbeuten erhalten.

Die höchste Ausbeute an Diaminodiethylenglykol mit 5 % wird gemäß WO 03/051508 A1 erhalten, wobei als Nebenprodukte 22% Morpholin und 36 % Monoaminodiethylenglykol entstehen.

Bei der Aminierung von Diethanolamin mit Ammoniak wird als Hauptprodukt Piperazin erhalten. Auch hier fällt das gewünschte lineare Diaminierungsprodukt Diethylentriamin nur in Spuren an, wenn hohe Diethanolaminumsätze gefahren werden.

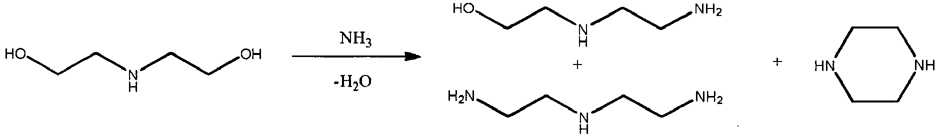

Bei der Umsetzung von Polyetherolen zu Polyetheraminen werden mit den vorstehend beschriebenen Verfahren aufgrund der bei der heterogen-katalysierten Aminierung herrschenden harschen Reaktionsbedingungen in hohem Ausmaß unerwünschte Nebenreaktionen zum dimeren sekundären Amin oder polymeren Kopplungsprodukt beobachtet, wie nachstehend anhand von Diethylenglykol verdeutlicht wird. Diese Nebenprodukte sind schwer vom gewünschten primären Diaminierungsprodukt abtrennbar. 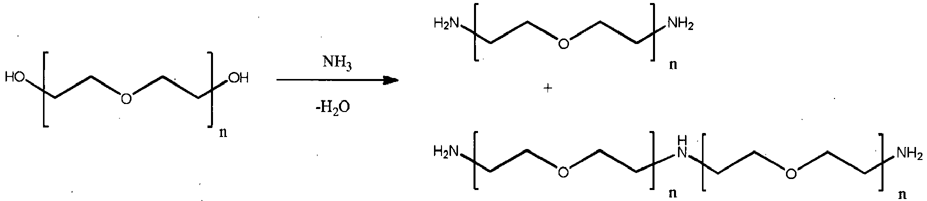 Ein weiteres Problem, das bei der Aminierung von Polyetherolen, insbesondere bei Polyethylen- und Polypropylenglykolderivaten, beobachtet wird, ist die Zersetzung dieser Ether bei den vorstehend beschriebenen Reaktionsbedingungen, da insbesondere die hohen Temperaturen und ein Wasserstoffstützdruck notwendig sind. Unter diesen Reaktionsbedingungen entstehen gasförmige Zersetzungsprodukte, die spezielle Sicherheitsvorkehrungen notwendig machen.

Ebenfalls nachteilig bei der heterogenen Katalyse der Aminierung von Alkoholen mit Ammoniak sind die dafür benötigten hohen Drücke.

Die homogen-katalysierte Aminierung von Alkoholen ist seit den 1970er Jahren bekannt, wobei meist Ruthenium- oder Iridiumkatalysatoren beschrieben sind. Die homogen-katalysierte Aminierung läuft, im Vergleich zu heterogen-katalysierten Reaktionen, bei deutlich niedrigeren Temperaturen von 100 bis 150°C ab. Die Umsetzung von Monoalkoholen mit primären und sekundären Aminen ist beispielsweise in folgenden Veröffentlichungen beschrieben: US 3,708,539; Y. Watanabe, Y. Tsuji, Y. Ohsugi, Tetrahedron Lett. 1981, 22, 2667-2670; S. Bähn, S. Imm, K. Mevius, L. Neubert, A. Tillack, J. M. J. Williams, M. Beller, Chem. Eur. J. 2010, DOI: 10.1002/chem.200903144; A. Tillack, D. Hollmann, D. Michalik, M. Beller, Tetrahedron Lett. 2006, 47, 8881-8885; D. Hollmann, S. Bähn, A. Tillack, M. Beller, Angew. Chem. Int. Ed. 2007, 46, 8291-8294; A. Tillack, D. Hollmann, K. Mevius, D. Michalik, S. Bahn, M. Beller, Eur. J. Org. Chem. 2008, 4745-4750; M. H. S. A. Hamid, C. L. Allen, G. W. Lamb, A. C. Maxwell, H. C. Maytum, A. J. A. Watson, J. M. J. Williams, J. Am. Chem. Soc. 2009, 131, 1766-1774; O. Saidi, A. J. Blacker, M. M. Farah, S. P. Marsden, J. M. J. Williams, Chem. Commun. 2010, 46, 1541-1543; EP 23 9943; N. Andrushko, V. Andrushko, P. Roose, K. Moonen, A. Börner, ChemCatChem, 2010, 2, 640-643; K. I. Fujita, R. Yamaguchi, Synlett, 2005, 4, 560-571; A. Tillack, D. Hollmann, D. Michalik, M. Beller, Tet. Lett. 2006, 47, 8881-8885; A. Del Zlotto, W. Baratta, M. Sandri, G. Verardo, P. Rigo, Eur. J. Org. Chem. 2004, 524-529; A. Fujita, Z. Li, N. Ozeki, R. Yamaguchi, Tetrahedron Lett. 2003, 44, 2687-2690; Y. Watanabe, Y. Morisaki, T. Kondo, T. Mitsudo J. Org. Chem. 1996, 61, 4214-4218, B. Blank, M. Madalska, R. Kempe, Adv. Synth. Catal. 2008, 350, 749-750, A. Martinez-Asencio, D. J. Ramon, M. Yus, Tetrahedron Lett. 2010, 51, 325-327. Der größte Nachteil der vorstehend beschriebenen Systeme ist, dass mit diesen Verfahren nur die Aminierung von Alkoholen mit primären und sekundären Aminen möglich ist. Die Umsetzung von Alkoholen mit Ammoniak zu primären Aminen, welches die wirtschaftlich attraktivste Aminierungsreaktion darstellt, wird in diesen Arbeiten nicht beschrieben. Auch wird kein Hinweis auf eine effiziente Rückführung der Edelmetallkatalysatoren gegeben.

Die homogen-katalysierte Aminierung von Alkoholen mit Ammoniak ist nur in wenigen Arbeiten beschrieben (WO 2010/018570). S. Imm, S. Bähn, L. Neubert, H. Neumann, M. Beller, Angew. Chem. 2010, 122, 8303-8306 und D. Pingen, C. Müller, D. Vogt, Angew. Chem. 2010, 122, 8307-8310 offenbaren die mit Rutheniumkatalysatoren homogen-katalysierte Aminierung von sekundären Alkoholen wie Cyclohexanol mit Ammoniak. Allerdings ist es mit den dort offenbarten Systemen nur möglich, sekundäre Alkohole mit Ammoniak zu aminieren. Die Aminierung von Di- und Triolen ist in diesen Arbeiten nicht beschrieben. Aus WO 2010/018570 und C. Gunanathan, D. Milstein, Angew. Chem. Int. Ed. 2008, 47, 8661-8664 ist die Aminierung von primären Alkoholen mit Ammoniak zu primären Monoaminen mit Hilfe von Ruthenium-Phosphan-Komplexen bekannt. Bei der Aminierung werden spezielle, acridinbasierte Pinzettenliganden eingesetzt. Die Reaktion wird bei Temperaturen von 110 bis 180 °C und NH₃-Drücken von bis zu 7,5 bar durchgeführt. Als Nebenprodukte bilden sich unter diesen Bedingungen bei Einsatz von primären Alkoholen hauptsächlich die entsprechenden Imine und Dialkylamine. Die Bildung von Dialkylaminen ist insbesondere bei der Aminierung von Diolen nachteilig, da sich unter diesen Bedingungen die zyklischen Amine bilden können, die ebenfalls unter die Gruppe der sekundären Amine fallen. Die Aminierung von Di- und Triolen mit Ammoniak ist nicht beschrieben.

R. Kawahara, K.I. Fujita, R. Yamaguchi, J. Am. Chem. Soc. DOI: 10.1021/ja107274w beschreibt die Aminierung primärer Monoalkohole und Triole mit Ammoniak unter Verwendung eines Iridiumkatalysators, der als Ligand Cp* (1,2,3,4,5-Pentamethylcyclopentadienyl) und Ammoniak aufweist. Mit dem dort beschriebenen Katalysatorsystem werden beim Umsatz primärer Monoalkohole mit Ammoniak jedoch ausschließlich die unerwünschten tertiären Aminen erhalten. Die Umsetzung von Glycerin mit Ammoniak führt ausschließlich zum unerwünschten bizyklischen Chinolizidin.

EP 0 234 401 A1 beschreibt die Umsetzung von Diethylenglykol mit Ammoniak in Gegenwart einer Rutheniumcarbonylverbindung. Bei dem in EP 0 234 401 A1 beschriebene Verfahren bilden sich lediglich das Monoaminierungsprodukt (Monoethanolamin), die sekundären und tertiären Amine (Di- und Triethanolamin) und zyklische Produkte (N-(Hydroxyethyl)piperazin und N,N'-Bis(hydroxyethyl)piperazin). Das gewünschte 1,2-Diaminoethan wird bei diesem Verfahren nicht erhalten.

Alle vorstehend beschriebenen Verfahren zur Umsetzung von Alkoholen mit Ammoniak haben den Nachteil, dass als Hauptprodukte nicht die gewünschten primären Amine gebildet werden. Zudem werden keine Konzepte zur Rückführung der teuren Edelmetallkatalysatoren beschrieben, was für eine technische Umsetzung dieser Verfahren aus ökonomischen Gründen jedoch erforderlich ist.

Aufgabe der vorliegenden Erfindung ist es, ein Verfahren zur Herstellung von primären Aminen durch Alkoholaminierung von Mono-, Di-, Tri- und Polyolen sowie von Alkanolaminen mit dem günstigen Aminierungsmittel Ammoniak bereitzustellen, bei dem der eingesetzte Katalysator abgetrennt und wiederverwendet werden kann. Die Umsetzung soll bei milderen Bedingungen ablaufen und höhere Selektivitäten bezüglich der Bildung primärer Amine liefern als die etablierten, heterogen katalytischen Umsetzungen. Insbesondere soll die Selektivität bei der Herstellung linearer primärer Amine sowie die Selektivität bei Herstellung von Di-, Tri- und Polyaminen verbessert werden.

Diese Aufgabe wird erfindungsgemäß gelöst durch das folgende Verfahren zur Herstellung von primären Aminen durch Alkoholaminierung von Alkoholen mit Ammoniak unter Wasserabspaltung umfassend die Schritte
(a) homogen-katalysiertes Umsetzen eines Reaktionsgemischs enthaltend mindestens einen Alkohol, Ammoniak, mindestens ein unpolares Lösungsmittel und mindestens einen Katalysator enthaltend mindestens ein Element ausgewählt aus den Gruppen 8, 9 und 10 des Periodensystems in der Flüssigphase, wobei ein Produktgemisch (P) erhalten wird,
(b) Phasentrennung des in Schritt (a) erhaltenen Produktgemischs (P) ggf. nach Temperaturerniedrigung, Druckerniedrigung und/oder Zugabe mindestens eines polaren Lösungsmittels, das mit dem unpolaren Lösungsmittel eine Mischungslücke aufweist, unter Erhalt mindestens einer polaren Produktphase (A) und mindestens einer unpolaren Phase (B) enthaltend mindestens einen Teil des eingesetzten Katalysators und Abtrennen der unpolaren Phase (B),
(c) Rückführen mindestens eines Teils der unpolaren Phase (B) in die Umsetzung in Schritt (a) und
(d) Abtrennen des Aminierungsprodukts aus der polaren Produktphase (A),
wobei das in (a) eingesetzte unpolare Lösungsmittel und der in Schritt (a) eingesetzte Katalysator so gewählt werden, dass sich der Katalysator in der unpolaren Phase (B) anreichert.

Überraschend wurde festgestellt, dass sich mit den im erfindungsgemäßen Verfahren eingesetzten Komplexkatalysatoren, die mindestens ein Element ausgewählt aus der Gruppe 8, 9 und 10 des Periodensystems enthalten, primäre Amine, bevorzugt Di-, Tri-und Polyamine sowie Alkanolamine durch die homogen-katalysierte Aminierung von Alkohlen mit Ammoniak unter Wasserabspaltung erhalten lassen. Das erfindungsgemäße Verfahren hat den Vorteil, dass es primäre Mono-, Di-, Tri- und Polyamine sowie Alkanolamine gegenüber den im Stand der Technik beschriebenen Verfahren in deutlich verbesserten Ausbeuten liefert. Darüber hinaus wird die Bildung unerwünschter Nebenprodukte wie sekundärer und tertiärer Amine sowie zyklischer Amine gegenüber dem Stand der Technik vermindert. Durch die entsprechende Wahl des Katalysators und der eingesetzten Lösungsmittel wird nach der Umsetzung ein flüssiges Zweiphasensystem erhalten, bei dem sich der Katalysator bevorzugt in der unpolaren Phase und das Aminierungsprodukt bevorzugt in der polaren Phase anreichert, so dass der Katalysator einfach mit der unpolaren Phase von der Produktphase abgetrennt und wiederverwertet werden kann.

### Edukte

Im erfindungsgemäßen Verfahren werden Alkohole als Edukte eingesetzt, die mindestens eine OH-Gruppe, bevorzugt in Form der funktionellen Gruppe der Formel (-CH₂-OH) (primäre Alkoholgruppe), oder (>CH-OH) (sekundäre Alkholgruppe) aufweisen. Bevorzugt weisen die Alkohole mindestens eine weitere funktionelle Gruppe (-X) auf, wobei (-X) ausgewählt ist aus Hydroxylgruppen (-OH) und primären Aminogruppen (-NH₂). Dabei werden im erfindungsgemäßen Verfahren besonders bevorzugt Edukte eingesetzt, in den (-X) ausgewählt ist aus der Gruppe der funktionellen Gruppen der Formeln (-CH₂-OH) und (>CH-OH) und (-CH₂-NH₂) und (>CH-NH₂). Die Edukte weisen dann mindestens eine funktionelle Einheit der Formel (-OH), bevorzugt der Formel (-CH₂-OH) und (>CH₂-OH) und/oder mindestens eine weitere funktionelle Gruppe ausgewählt aus der Gruppe der funktionellen Gruppen der Formel (-CH₂-OH) und (>CH₂-OH) und (-CH₂-NH₂) und (>CH₂-NH₂) auf. Ganz besonders bevorzugt werden erfindungsgemäß die vorstehend beschriebenen Edukte eingesetzt, die bei Umsetzung mit NH₃ lineare primäre Amine ergeben, ganz besonders bevorzugt werden lineare Diole eingesetzt, die mindestens 2 OH-Gruppen aufweisen, das heißt zwei primäre und/oder sekundäre Alkoholgruppen aufweisen sowie lineare Alkanolamine, die mindestens eine primäre oder sekundäre Alkoholgruppe in Form von (-CH₂-OH) oder (>CH-OH) aufweisen.

Als Edukte sind praktisch alle Alkohole geeignet, die die vorstehend genannten Voraussetzungen erfüllen. Die Alkohole können linear, verzweigt oder zyklisch, vorzugsweise linear sein. Die Alkohole können darüber hinaus Substituenten tragen, die sich unter den Reaktionsbedingungen der Alkoholaminierung inert verhalten, beispielsweise Alkoxy, Alkenyloxy, Alkylamino, Dialkylamino und Halogen (F, Cl, Br, I).

Geeignete Edukte, die im erfindungsgemäßen Verfahren eingesetzt werden können, sind beispielsweise Monoalkohole, Diole, Triole, Polyole und Alkanolamine, die mindestens eine OH-Gruppe, bevorzugt in Form der funktionellen Gruppen der Formel (-CH₂-OH) oder (>CH-OH) und mindestens eine weitere funktionelle Gruppe (-X) aufweisen, wobei (-X) ausgewählt ist aus Hydroxylgruppen und primären Aminogruppen.

Darüber hinaus sind Diole, Triole, Polyole und Alkanolamine geeignet, die mindestens eine OH-Gruppe, und mindestens eine weitere funktionelle primäre oder sekundäre OH-Einheit oder NH₂-Einheit aufweisen.

Als Edukte können alle bekannten Diole eingesetzt werden, die mindestens eine primäre oder sekundäre OH-Gruppe aufweisen. Beispiele für Diole, die als Edukte in das erfindungsgemäße Verfahren eingesetzt werden können, sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), 1,5-Pentandiol, 1,2-Pentandiol, 1,6-Hexandiol, 1,2-Hexandiol, 1,7-Heptandiol, 1,2-Heptandiol, 1,8-Oktandiol, 1,2-Oktandiol, 1,9-Nonandiol, 1,2-Nonandiol, 2,4-Dimethyl-2,5-hexandiol, Hydroxypivalinsäureneopentylglykolester, Diethylenglykol, Triethylenglykol, 2-Buten-1,4-diol, 2-Butin-1,4-diol, Poylyethlenglykole, Polypropylenglykole wie 1,2-Polypropylenglykol und 1,3 Polypropylenglykol, Polytetrahydrofuran, Diethanolamin, 1,4-Bis-(2-hydroxyethyl)piperazin, Diisopropanolamin, N-Butyldiethanolamin, 1,10-Decandiol, 1,12-Dodecandiol, 2,5-(Dimethanol)-furan, 1,4-Bis(hydroxymethyl)cyclohexan, C₃₆-Diol (Gemisch aus Isomeren von Alkoholen der Summenformel (C₃₆H₇₄O₂)) und N-Methyldiethanolamin, Isosorbid (1,4:3,6 - Dianhydroglucitol), Isomannit (1,4 : 3,6 -Dianhydromannitol, Düsopropanol-p-Toluidin, N,N-Di-(2-hydroxyethyl)aniline, Diisopronanolamin. 2,5-(Dimethanol)-furan wird auch als 2,5-Bis(hydroxymethyl)furan bezeichnet.

Bevorzugt sind Diole, die zwei funktionelle Gruppen der Formel (-CH₂-OH) aufweisen.

Insbesondere bevorzugte Diole sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 2-Methyl-1,3-propandiol, 2,2-Dimethyl-1,3-propandiol (Neopentylglykol), 1,5-Pentandiol, 1,6-Hexandiol, 1,7-Heptandiol, 1,8-Oktandiol, 1,9-Nonandiol, Diethylenglykol, Triethylenglykol, Poylyethlenglykole, Polypropylenglykole wie 1,2-Polypropylenglykol und 1,3 Polypropylenglykol, Polytetrahydrofuran, Diethanolamin, Diisopropanolamin, N-Butyldiethanolamin, 2,5-(Dimethanol)-furan und N-Methyldiethanolamin.

Als Edukte können alle bekannten Triole eingesetzt werden, bevorzugt werden Triole, die mindestens eine funktionelle Gruppe der Formel (-CH₂-OH) oder (>CH-OH) aufweisen, besonders bevorzugt Triole mit mindestens zwei funktionellen Gruppen der Formel (-CH₂-OH) oder (>CH-OH). Beispiele für Triole, die als Edukte in das erfindungsgemäße Verfahren eingesetzt werden können, sind Glycerin, Trimethylolpropan, Triisopropanolamin und Triethanolamin.

Besonders bevorzugte Triole sind Glycerin, Trimethylolpropan und Triethanolamin.

Als Edukte können alle bekannten Polyole eingesetzt werden, bevorzugt enthalten diese mindestens eine funktionelle Gruppe der Formel (-CH₂-OH) oder (>CH-OH). Beispiele für Polyole, die als Edukte in dem erfindungsgemäßen Verfahren eingesetzt werden können, sind Polyvinylalkohol, 2,2-Bis(hydroxymethyl)-1,3-propandiol (Pentaerythrit), Sorbit, Inositol, Zucker und Polymere wie beispielsweise Glucose, Mannose, Fructose, Ribose, Desoxyribose, Galactose, N-Acetyl-Glucosamin, Fucose, Rhamnose, Saccharose, Lactose, Cellobiose, Maltose und Amylose, Cellulose, Stärke und Xanthan.

Bevorzugt sind Polyole, die mindestens zwei funktionelle Gruppen der Formel (-CH₂-OH) oder (>CH-OH) aufweisen.

Insbesondere bevorzugte Polyole sind Glucose und Cellulose.

Als Edukte können auch alle bekannten Alkanolamine eingesetzt werden, die mindestens eine OH-Gruppe, bevorzugt eine primäre oder sekundäre Hydroxylgruppe und mindestens eine primäre Aminogruppe (-NH₂) aufweisen. Die Alkanolamine werden im Rahmen der Erfindung mit unter die als Edukte einzusetzenden Alkohole gefasst. Beispiele für Alkanolamine, die als Edukte in das erfindungsgemäße Verfahren eingesetzt werden können, sind Monoaminoethanol, 3-Aminopropan-1-ol, 2-Aminopropan-1-ol, 4-Aminobutan-1-ol, 2-Aminobutan-1-ol, 3-Aminobutan-1-ol, 5-Aminopentan-1-ol, 2-Aminopentan-1-ol, 6-Aminohexan-1-ol, 2-Aminohexan-1ol, 7-Aminoheptan-1-ol, 2-Aminoheptan-1-ol, 8-Aminooctan-1-ol, 2-Aminooctan-1-ol, N-(2-Hydroxyethyl)anilin, 2-(2-Aminoethoxy)ethanol, N-(2-Hydroxyethyl)-1,3-propandiamin und Aminodiethylenglykol (2-(2-Aminoethoxy)ethanol).

Bevorzugt sind Alkanolamine, die mindestens eine primäre Hydroxylgruppe (-CH₂-OH) und mindestens eine primäre Aminogruppe der Formel (-CH₂-NH₂) aufweisen.

Insbesondere bevorzugte Alkanolamine sind Monoaminoethanol, 3-Aminopropan-1-ol, 2-Aminopropan-1-ol, 4-Aminobutan-1-ol und 2-(2-Aminoethoxy)ethanol.

### Komplexkatalysator

Im erfindungsgemäßen Verfahren wird mindestens ein Komplexkatalysator, der mindestens ein Element ausgewählt aus den Gruppen 8, 9 und 10 des Periodensystems (Nomenklatur gemäß IUPAC) enthält, eingesetzt. Die Elemente der Gruppe 8, 9 und 10 des Periodensystems umfassen Eisen, Cobalt, Nickel, Ruthenium, Rhodium, Palladium, Osmium, Iridium und Platin. Bevorzugt sind Komplexkatalysatoren, die mindestens ein Element ausgewählt aus Ruthenium und Iridium enthalten.

Der Komplexkatalysator enthält bevorzugt mindestens einen Donorliganden, insbesondere einen Phosphordonorliganden. Besonders bevorzugt enthält der Komplexkatalysator mindestens ein Element ausgewählt aus Ruthenium und Iridium und mindestens einen Phosphordonorliganden.

In einer Ausführungsform wird das erfindungsgemäße Verfahren in Gegenwart mindestens eines Komplexkatalysators der allgemeinen Formel (I) durchgeführt: 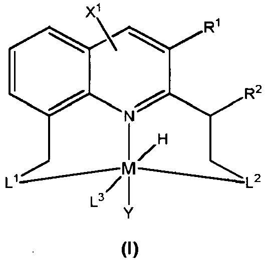 in der
- L¹ und L²: unabhängig voneinander Phosphin (PR^{a}R^{b}), Amin (NR^{a}R^{b}), Sulfid, SH, Sulfoxid (S(=O)R), C₅-C₁₀-Heteroaryl enthaltend mindestens ein Heteroatom ausgewählt aus Stickstoff (N), Sauerstoff (O) und Schwefel (S), Arsin (AsR^{a}R^{b}), Stiban (SbR^{a}R^{b}) oder N-heterozyklische Carbene der Formel (II) oder (III): 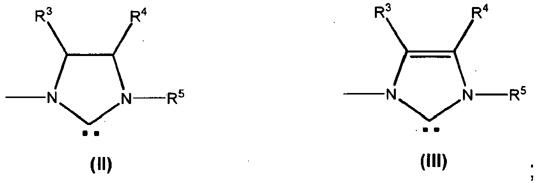
- L³: einzähniger Zweielektronendonor ausgewählt aus der Gruppe Kohlenmonoxid (CO), PR^{a}R^{b}R^{c}, NO⁺, AsR^{a}R^{b}R^{c}, SbR^{a}R^{b}R^{c}, SR^{a}R^{b}, Nitril (RCN), Isonitril (RNC), Stickstoff (N₂), Phosphortrifluorid (PF₃), Kohlenstoffmonosulfid (CS), Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten.

Hierbei ist darauf hinzuweisen, dass der Komplexkatalysator der Formel (I) für den Fall, dass Y ein neutrales Molekül aus der Gruppe NH₃, NR₃, R₂NSO₂R ist, eine positive Ladung trägt.

In einer bevorzugten Ausführungsform wird das erfindungsgemäßen Verfahrens in Gegenwart mindestens eines homogen gelösten Komplexkatalysators der Formel (I) durchgeführt, wobei die Substituenten folgende Bedeutung haben:
- L¹ und L²,: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S;
- L³: einzähniger Zweielektronendonor, ausgewählt aus der Gruppe CO, PR^{a}R^{b}R^{c}, NO⁺, RCN, RNC, N₂, PF₃, CS, Pyridin, Thiophen und Tetrahydrothiophen;
- R¹ und R²: beide Wasserstoff oder gemeinsam mit den Kohlenstoffatomen, an die sie gebunden sind ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes C₁-C₁₀-Alkyl, C₃-C₁₀)-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S;
- Y: monoanionischer Ligand ausgewählt aus der Gruppe H, F, Cl, Br, OCOR, OCOCF₃, OSO₂R, OSO₂CF₃, CN, OH, OR oder N(R)₂;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium.

In einer weiteren bevorzugten Ausführungsform wird das erfindungsgemäße Verfahren in Gegenwart mindestens eines homogen gelösten Komplexkatalysator durchgeführt, wobei R¹ und R² beide Wasserstoff sind und der Komplexkatalysator ein Katalysator der Formel (IV) ist: 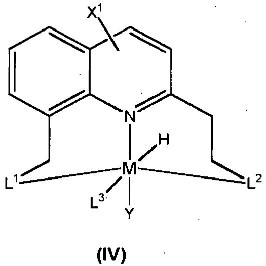 und X¹, L¹, L², L³ und Y die vorstehend genannten Bedeutungen aufweisen.

In einer weiteren bevorzugten Ausführungsform wird das erfindungsgemäßen Verfahrens in Gegenwart mindestens eines homogen gelösten Komplexkatalysator durchgeführt, wobei R¹ und R² gemeinsam mit den Kohlenstoffatomen, an die sie gebunden sind ein Phenylring bilden, der zusammen mit der Chinolinyleinheit der Formel (I) eine Acridinyleinheit bildet und der Komplexkatalysator ein Katalysator der Formel (V) ist: 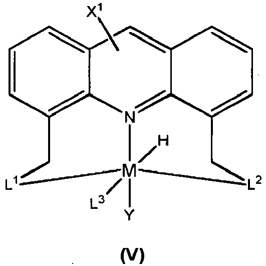 und X¹, L¹, L², L³ und Y die vorstehend genannten Bedeutungen aufweisen.

Nachfolgend werden exemplarisch einige Komplexkatalysatoren (Formeln (VI), (VII), (VIII), (IX), (X), (XI), (XII) und (XIII)) aufgeführt, die im erfindungsgemäßen Verfahren eingesetzt werden können:

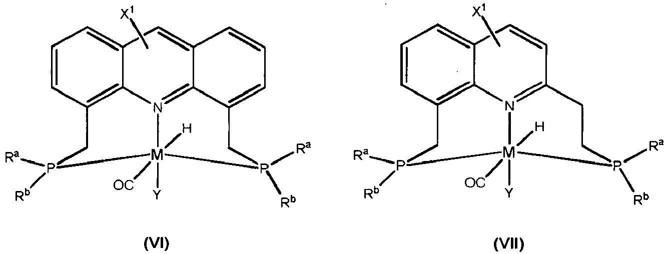

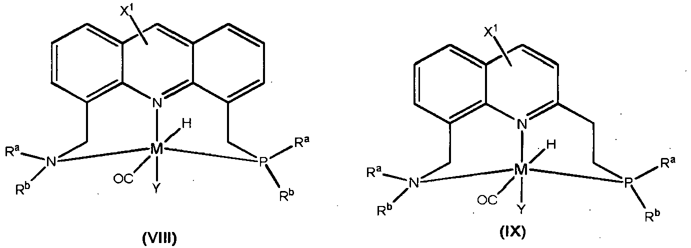

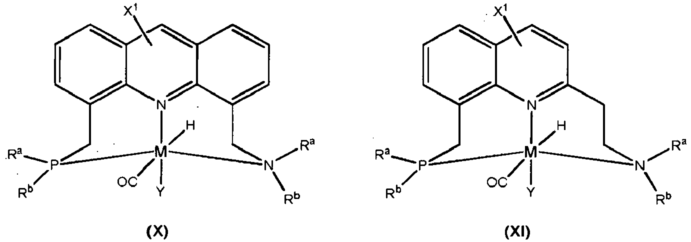

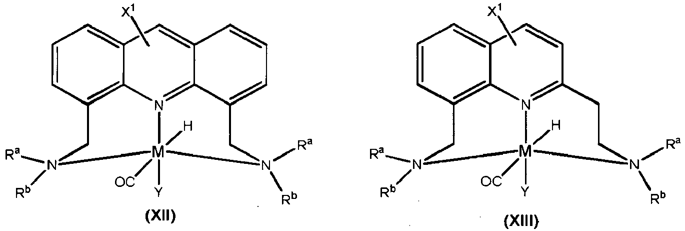

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, OCOR, OCOCF₃, OSO₂R, OSO₂CF₃, CN, OH, OR, N(R)₂;
- R: unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl, C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- X¹: ein, zwei oder drei, Substituenten an einem oder mehreren Atomen der Acridinyleinheit oder ein oder zwei Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, - C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert.-Butyl, Cyclohexyl, Cyclopentyl, Phenyl oder Mesityl;
- Y: monoanionischer Ligand ausgewählt aus der Gruppe H, F, Cl, Br, OCOCH₃, OCOCF₃, OSO₂CF₃, CN und OH;
- X¹: ein Substituent an einem Atom der Acridinyleinheit oder ein Substituenten an einem Atom der Chinolinyleinheit,
wobei X¹ ausgewählt ist aus der Gruppe bestehend aus Wasserstoff, F, Cl, Br, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert. Butyl, Cyclohexyl, Cyclopentyl, Phenyl oder Mesityl;
- Y: monoanionischer Ligand aus der Gruppe F, Cl, Br, I, OCOCH₃, OCOCF₃, OSO₂CF₃, CN und OH;
- X¹: Wasserstoff;
und
- M: Ruthenium oder Iridium
bedeuten.

In einer besonders bevorzugten Ausführungsform ist L³ Kohlenmonoxid (CO).

In einer besonders bevorzugten Ausführungsform wird das erfindungsgemäße Verfahrens in Gegenwart eines Komplexkatalysators der Formel (XIVa) durchgeführt:

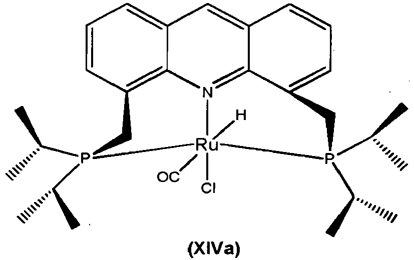

In einer insbesondere bevorzugten Ausführungsform wird das erfindungsgemäße Verfahren in Gegenwart eines Komplexkatalysators der Formel (XIVb) durchgeführt:

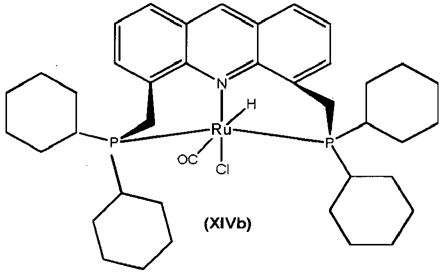

In einer weiteren besonders bevorzugten Ausführungsform wird das erfindungsgemäße Verfahrens in Gegenwart mindestens eines homogen gelösten Komplexkatalysator der Formel (XV) durchgeführt, in der R¹, R², R³, L¹, L² und L³ die vorstehend beschriebenen Bedeutungen haben.

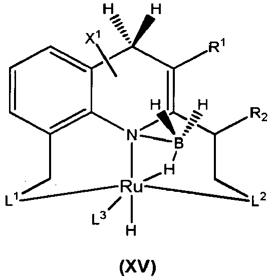

Komplexkatalysatoren der Formel (XV) sind erhältlich durch Umsetzung von Katalysatoren der Formel (I) mit Natriumborhydrid (NaBH₄). Die Umsetzung folgt dabei der allgemeinen Reaktionsgleichung:

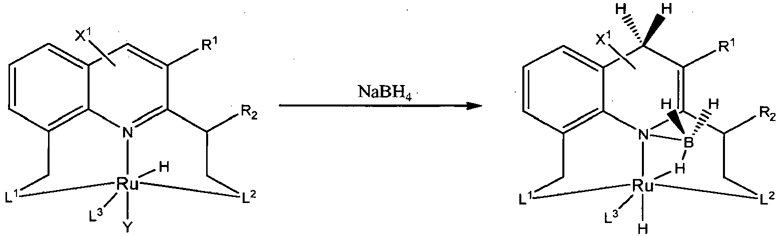

In einer weiteren besonders bevorzugten Ausführungsform wird das erfindungsgemäße Verfahrens in Gegenwart eines Komplexkatalysators der Formel (XVIa) durchgeführt:

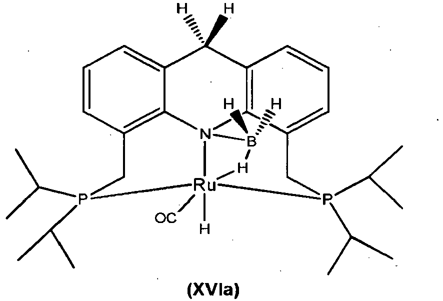

In einer weiteren besonders bevorzugten Ausführungsform wird das erfindungsgemäße Verfahren in Gegenwart eines Komplexkatalysators der Formel (XVIb) durchgeführt:

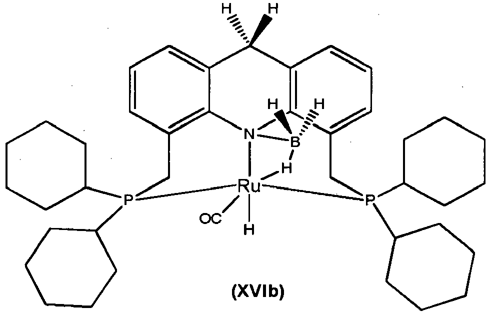

Das Boranderivat der Formel (XVIa) ist gemäß folgender Reaktionsgleichung zugänglich:

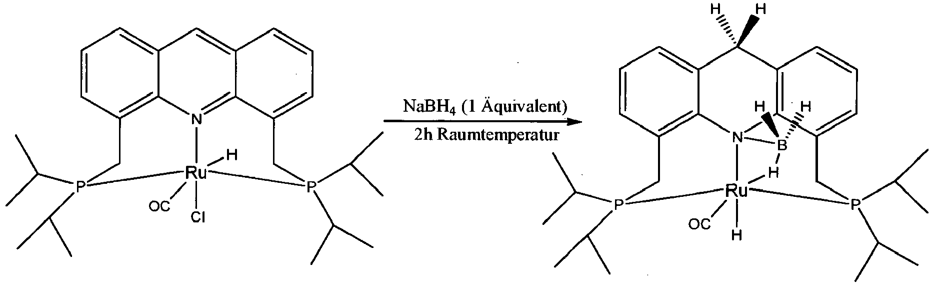

Das Boranderivat der Formel (XVIb) ist gemäß folgender Reaktionsgleichung zugänglich:

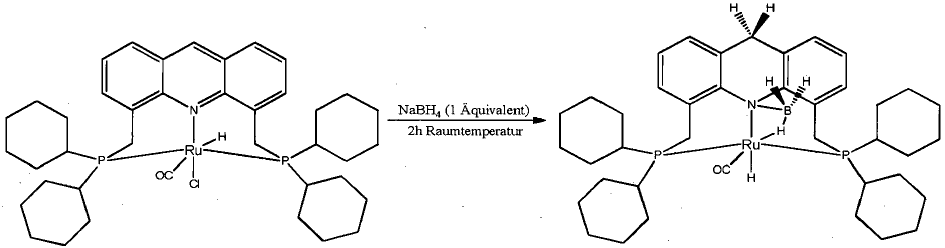

Im Rahmen der vorliegenden Erfindung werden unter C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C₁₀-Dialkylamino, C₁₀-C₁₂-Diarylamino, C₁₀-C₂₀Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.

Unter C₃-C₁₀-Cycloalkyl werden vorliegend gesättigte, ungesättigte monozyklische und polyzyklische Gruppen verstanden. Beispiele für C₃-C₁₀-Cycloalkyl sind Cyclopropyl, Cyclobutyl, Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Im Rahmen der vorliegenden Erfindung wird unter C₅-C₁₀-Aryl ein aromatisches Ringsystem mit 5 bis 10 Kohlenstoffen verstanden. Das aromatisches Ringsystem kann monozyklisch oder bizyklisch sein. Beispiele für Arylgruppen sind Phenyl, Naphthyl wie 1-Naphthyl und 2-Naphthyl. Die Arylgruppe kann unsubstituiert oder substituiert sein, mit einem oder mehreren Substituenten wie vorstehend unter C₁-C₁₀-Alkyl definiert.

Im Rahmen der vorliegenden Erfindung wird unter C₅-C₁₀-Heteroaryl ein heteroaromatisches System verstanden, das mindestens ein Heteroatom, ausgewählt aus der Gruppe N, O und S, enthält. Die Heteroarylgruppen können monozyklisch oder bizyklisch sein. Für den Fall, dass Stickstoff ein Ringatom ist, umfasst die vorliegende Erfindung auch N-Oxide der stickstoffenthaltenden Heteroaryle. Beispiele für Heteroaryle sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₀-Alkyl definiert wurden.

Im Rahmen der vorliegenden Erfindung werden unter C₃-C₁₀-Heterocyclyl fünf- bis zehngliedrige Ringsysteme verstanden, die mindestens ein Heteroatom aus der Gruppe N, O und S enthalten. Die Ringsysteme können mono- oder bizyklisch sein. Beispiele für geeignete heterozyklische Ringsysteme sind Piperidinyl-, Pyrrolidinyl, Pyrrolinyl, Pyrazolinyl, Pyrazolidinyl, Morpholinyl, Thiomorpholinyl, Pyranyl, Thiopyranyl, Piperazinyl, Indolinyl, Dihydrofuranyl, Tetrahydrofuranyl, Dihydrothiophenyl, Tetrahydrothiophenyl, Dihydropyranyl und Tetrahydropyranyl.

In einer weiteren Ausführungsform wird im erfindungsgemäßen Verfahren mindestens ein Komplexkatalysator, der mindestens ein Element ausgewählt aus den Gruppen 8, 9 und 10 des Periodensystems (Nomenklatur gemäß IUPAC) sowie mindestens einen Phosphordonorliganden der allgemeinen Formel (XXI) enthält, 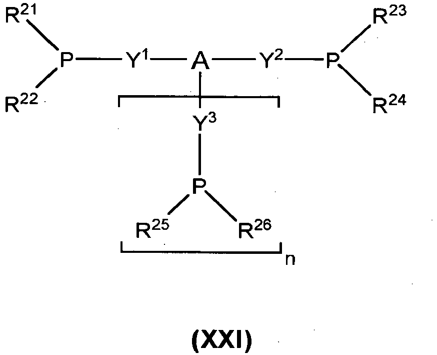 eingesetzt, wobei
- n: ist 0 oder 1;
- R²¹, R²², R²³, R²⁴, R²⁵, R²⁶: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₅-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
   wobei R²⁷ ausgewählt ist aus C₁-C₁₀-Alkyl und C₅-C₁₀-Aryl;
   oder
ii) eine Brückengruppe der Formel (XXII) oder (XXIII): 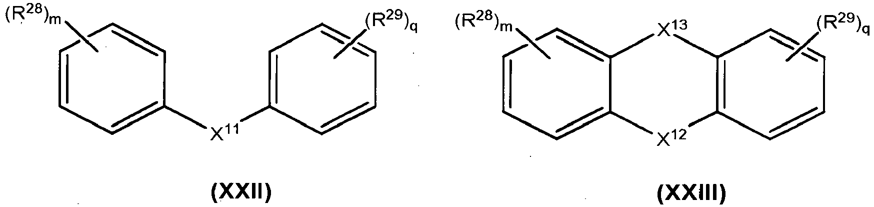
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S;
- X¹³: ist eine Bindung, NH, NR³⁰, O, S oder CR³¹R³²;
- R³⁰: ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- R³¹, R³²: sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

A ist erfindungsgemäß eine Brückengruppe. Für den Fall, dass A ausgewählt ist aus der Gruppe unsubstituiertes oder zumindest monosubstituiertes C₁-C₆-Alkan, C₃-C₁₀-Cycloalkan, C₃-C₁₀ Heterocycloalkan, C₅-C₁₄-Aromat und C₅-C₆-Heteroaromat und Brückengruppen der Formel (II) oder (III), sind für den Fall (n = 0) zwei Wasserstoffatome der Brückengruppe durch Bindungen zu den benachbarten Substituenten Y¹ und Y² ersetzt. Für den Fall (n = 1) sind drei Wasserstoffatome der Brückengruppe durch drei Bindungen zu den benachbarten Substituenten Y¹, Y² und Y³ ersetzt.

Für den Fall, dass A P (Phosphor) ist, bildet der Phosphor für den Fall (n=0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n=1) bildet der Phosphor drei Bindungen zu den benachbarten Substituenten Y¹, Y² und Y³ aus.

Für den Fall, dass A N (Stickstoff) ist, bildet der Stickstoff für den Fall (n = 0) zwei Bindungen zu den benachbarten Substituenten Y¹ und Y² und eine Bindung zu einem Substituenten ausgewählt aus der Gruppe bestehend aus C₁-C₄-Alkyl und Phenyl aus. Für den Fall (n = 1) bildet der Stickstoff drei Bindungen zu den benachbarten Substituenten Y¹, Y² und Y³ aus.

Für den Fall, dass A O (Sauerstoff) ist, ist n = 0. Der Sauerstoff bildet zwei Bindungen zu den benachbarten Substituenten Y¹ und Y² aus.

Bevorzugt sind Komplexkatalysatoren, die mindestens ein Element ausgewählt aus Ruthenium und Iridium enthalten.

In einer bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXI) enthält, durchgeführt, wobei
- n: ist 0 oder 1;
- R²¹, R²², R²³, R²⁴, R²⁵, R²⁶: sind unabhängig voneinander unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
- A: ist
i) eine Brückengruppe ausgewählt aus der Gruppe unsubstituiertes C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S;
   oder
ii) eine Brückengruppe der Formel (XXII) oder (XXIII): 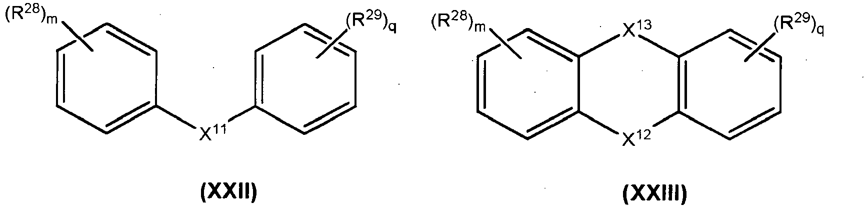
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S;
- X¹³: ist eine Bindung, NH, NR³⁰, O, S oder CR³¹R³²;
- R³⁰: ist unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S;
- R³¹, R³²: sind unabhängig voneinander unsubstituiertes C₁-C₁₀-Alkyl, C₁-C₁₀-Alkoxy, C₃-C₁₀-Cycloalkyl, C₃-C₁₀-Cycloalkoxy,C₃-C₁₀-Heterocyclyl enthaltend mindestens ein Heteroatom ausgewählt
aus N, O und S, C₅-C₁₄-Aryl, C₅-C₁₄-Aryloxy oder C₅-C₁₀-Heteroaryl enthaltend mindestens ein Heteroatom ausgewählt aus N, O und S;
- Y¹, Y², Y³: sind unabhängig voneinander eine Bindung, unsubstituiertes Methylen, Ethylen, Trimethylen, Tetramethylen, Pentamethylen oder Hexamethylen.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXV) enthält, 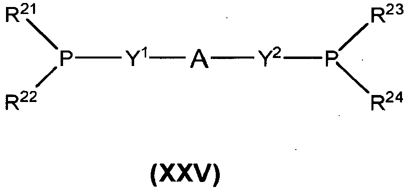 durchgeführt, wobei
- R²¹, R²², R²³, R²⁴: sind unabhängig voneinander unsubstituiertes oder zumindest monosubstituiertes C₁-C₁₀-Alkyl, C₁-C₄-Alkyldiphenylphosphin (-C₁-C₄-Alkyl-P(Phenyl)₂), C₃-C₁₀-Cycloalkyl, C₃-C₁₀-Heterocyclyl, enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist
i) eine Brückengruppe ausgewählt aus der Gruppe unsubstituiertes oder zumindest monosubstituiertes N, O, P, C₁-C₆-Alkan, C₃-C₁₀-Cycloalkan, C₃-C₁₀-Heterocycloalkan enthaltend mindestens ein Heteroatom ausgewählt aus N, O und S, C₅-C₁₄-Aromat und C₅-C₆-Heteroaromat enthaltend mindestens ein Heteroatom ausgewählt aus N, O und S,
   wobei die Substituenten ausgewählt sind aus der Gruppe bestehend aus:
   C₁-C₄-Alkyl, Phenyl, F, Cl, Br, OH, OR²⁷, NH₂, NHR²⁷ oder N(R²⁷)₂,
   wobei R²⁷ ausgewählt ist aus C₁-C₁₀-Alkyl und C₅-C₁₀-Aryl;
   oder
ii) eine Brückengruppe der Formel (XXII) oder (XXIII): 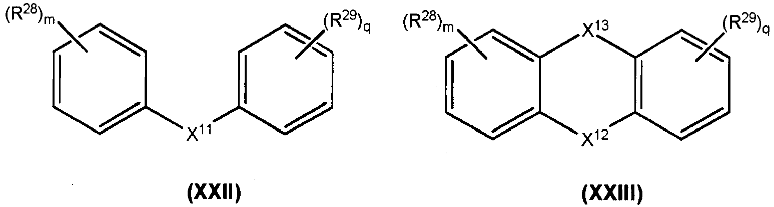
- m, q: sind unabhängig voneinander 0, 1, 2, 3 oder 4;
- R²⁸, R²⁹: sind unabhängig voneinander ausgewählt aus der Gruppe C₁-C₁₀-Alkyl, F, Cl, Br, OH, OR²⁷, NH₂, NHR²⁷ und N(R²⁷)₂,
wobei R²⁷ ausgewählt ist aus C₁-C₁₀-Alkyl und C₅-C₁₀-Aryl;
- X¹¹, X¹²: sind unabhängig voneinander NH, O oder S,
- X¹³: ist eine Bindung, NH, NR³⁰, O, S oder CR³¹R³²;
- R³⁰: ist unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- R³¹, R³²: sind unabhängig voneinander unsubstituiertes oder zumindest monosubstituiertes C₁-C₁₀-Alkyl, C₁-C₁₀-Alkoxy, C₃-C₁₀-Cycloalkyl, C₃-C₁₀-Cycloalkoxy, C₃-C₁₀-Heterocyclyl enthaltend mindestens ein Heteroatom ausgewählt aus N, O und S, C₅-C₁₄-Aryl, C₅-C₁₄-Aryloxy oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y¹, Y²: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXVI) enthält, 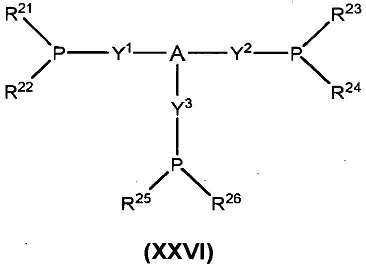 durchgeführt, wobei
- R²¹, R²², R²³, R²⁴, R²⁵, R²⁶: sind unabhängig voneinander unsubstituiertes oder zumindest monosubstituiertes C₁-C₁₀-Alkyl, C₁-C₄-Alkyldiphenylphosphin, C₃-C₁₀-Cycloalkyl, C₃-C₁₀-Heterocyclyl enthaltend mindestens ein Heteroatom ausgewählt aus N, O und S, C₅-C₁₄-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- A: ist eine Brückengruppe ausgewählt aus der Gruppe unsubstituierte oder zumindest monosubstituiertes N, P, C₁-C₆-Alkan, C₃-C₁₀-Cycloalkan, C₃-C₁₀-Heterocycloalkan enthaltend mindestens ein Heteroatom ausgewählt
aus N, O und S, C₅-C₁₄-Aromat und C₅-C₆-Heteroaromat enthaltend mindestens ein Heteroatom ausgewählt aus N, O und S,
wobei die Substituenten ausgewählt sind aus der Gruppe bestehend aus: C₁-C₄-Alkyl, Phenyl, F, Cl, Br, OH, OR²⁷, NH₂, NHR²⁷ oder N(R²⁷)₂,
wobei R²⁷ ausgewählt ist aus C₁-C₁₀-Alkyl und C₅-C₁₀-Aryl;
- Y¹, Y², Y³: sind unabhängig voneinander eine Bindung, unsubstituiertes oder zumindest monosubstituiertes Methylen, Ethylen, Trimethylen, Tetramethylen, Pentamethylen oder Hexamethylen,
wobei die Substituenten ausgewählt sind aus der Gruppe bestehend aus: F, Cl, Br, OH, OR²⁷, CN, NH₂, NHR²⁷, N(R²⁷)₂ und C₁-C₁₀-Alkyl,
wobei R²⁷ ausgewählt ist aus C₁-C₁₀-Alkyl und C₅-C₁₀-Aryl.

In einer weiteren bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXV) enthält, durchgeführt, wobei
- R²¹, R²², R²³, R²⁴: sind unabhängig voneinander Methyl, Ethyl, Isopropyl, tert.-Butyl, Cyclopentyl, Cyclohexyl, Phenyl, oder Mesityl;
- A: ist
i) eine Brückengruppe ausgewählt aus der Gruppe Methan, Ethan, Propan, Butan, Cyclohexan, Benzol, Napthalin und Anthracen;
   oder
ii) eine Brückengruppe der Formel (XXVII) oder (XXVIII): 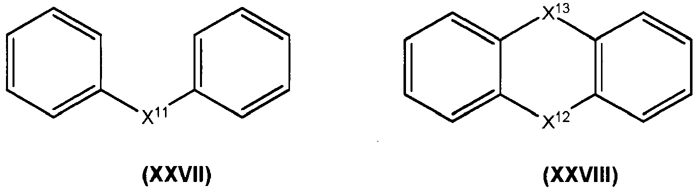
- X¹¹, X¹²: sind unabhängig voneinander NH, O oder S;
- X¹³: ist eine Bindung, NH, O, S oder CR³¹R³²;
- R³¹, R³²: sind unabhängig voneinander unsubstituiertes C₁-C₁₀-Alkyl;
- Y¹, Y²: sind unabhängig voneinander eine Bindung, Methylen oder Ethylen.

In einer besonders bevorzugten Ausführungsform wird das erfindungsgemäße Verfahren in Gegenwart mindestens eines Komplexkatalysators, der mindestens ein Element ausgewählt aus den Gruppen 8, 9 und 10 des Periodensystems sowie mindestens einen Phosphordonorliganden der allgemeinen Formel (XXIX) oder (XXX) enthält, 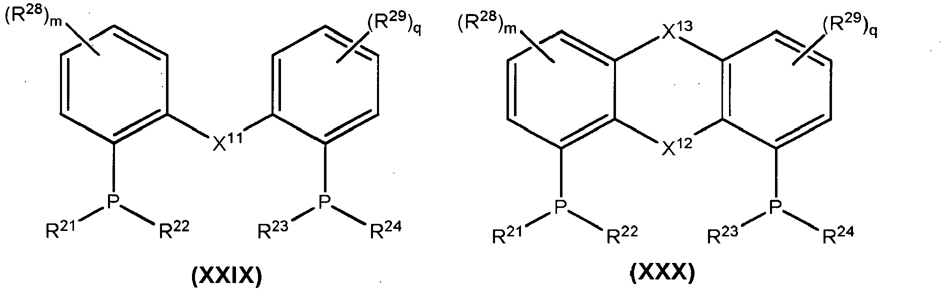 durchgeführt, wobei für m, q, R²¹, R²², R²³, R²⁴, R²⁸, R²⁹, X¹⁹, X¹² und X¹³ die vorstehend aufgeführten Definitionen und Bevorzugungen gelten.

In einer weiteren besonders bevorzugten Ausführungsform wird das erfindungsgemäße Verfahren in Gegenwart mindestens eines Komplexkatalysators, der mindestens ein Element ausgewählt aus der Gruppe Ruthenium und Iridium sowie mindestens einen Phosphordonorliganden ausgewählt aus der Gruppe 1,2-Bis(diphenylphosphino)ethan (dppe), 1,3-Bis(diphenylphosphino)propan (dppp), 1,4-Bis(diphenylphosphino)butan (dppb), 2,3-Bis(dicyclohexylphosphino)ethan (dcpe), 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält, durchgeführt.

In einer weiteren besonders bevorzugten Ausführungsform wird das erfindungsgemäße Verfahren in Gegenwart eines Komplexkatalysators, der Ruthenium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält, durchgeführt.

In einer weiteren besonders bevorzugten Ausführungsform wird das erfindungsgemäße Verfahren in Gegenwart eines Komplexkatalysators, der Iridium sowie mindestens einen Phosphordonorliganden ausgewählt aus der Gruppe 4,5-Bis(diphenylphosphino)-9,9-dimethylxanthen (Xantphos), Bis(2-diphenylphosphinoethyl)phenylphosphin und 1,1,1-Tris(Diphenylphosphinomethyl)ethan (Triphos) enthält, durchgeführt.

Im Rahmen der vorliegenden Erfindung werden unter C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀)-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C₁₀-Dialkylamino, C₁₀-C₁₂-Diarylamino, C₁₀-C₂₀Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.

Die vorstehende Definition für C₁-C₁₀-Alkyl gilt für C₁-C₃₀-Alkyl und für C₁-C₆-Alkan sinngemäß.

Unter C₃-C₁₀-Cycloalkyl werden vorliegend gesättigte, ungesättigte monozyklische und polyzyklische Gruppen verstanden. Beispiele für C₃-C₁₀-Cycloalkyl sind Cyclopropyl, Cyclobutyl, Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Die vorstehend genannte Definition für C₃-C₁₀-Cycloalkyl gilt für C₃-C₁₀-Cycloalkan sinngemäß.

Die homogenen Katalysatoren können sowohl direkt in ihrer aktiven Form als auch ausgehend von üblichen Vorstufen unter Zugabe der entsprechenden Liganden erst unter den Reaktionsbedingungen erzeugt werden. Übliche Vorstufen sind beispielsweise [Ru(p-cymene)Cl₂]₂, [Ru(benzol)Cl₂]ₙ, [Ru(CO)₂Cl₂]ₙ, [Ru(CO)₃Cl₂]₂ [Ru(COD)(allyl)], [RuCl₃*H₂O], [Ru(acteylacetonat)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(cyclopentadienyl)(PPh₃)₂Cl], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcylcopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(binap)Cl₂], [Ru(bipyridin)₂Cl₂*2H₂O], [Ru(COD)Cl₂]₂, [Ru(pentamethylcyclopentadienyl)(COD)CI], [Ru₃(CO)₁₂], [Ru(tetraphenylhydroxy-cyclopentadienyl)(CO)₂H], [Ru(PMe₃)₄(H)_{2]}, [Ru(PEt₃)₄(H)₂], [Ru(P*n*Pr₃)₄(H)₂], [Ru(P*n*Bu₃)₄(H)₂], [Ru(P*n*Octyl₃)₄(H)₂], [IrCl₃*H₂O], KIrCl₄, K₃lrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂, [Ir(cylopentadienyl)(CO)₂], [Ir(pentamethylcyclopentadienyl)(CO)₂], [Ir(PPh₃)₂(CO)(H)], [Ir(PPh₃)₂(CO)(Cl)], [Ir(PPh₃)₃(Cl)].

### Alkoholaminierung (Schritt (a))

Die Alkoholaminierung in Schritt (a) erfolgt durch homogen-katalysierte Umsetzung eines oder mehrere Hydroxylgruppen enthaltender Edukte mit Ammoniak in Gegenwart mindestens eines der vorstehend beschriebenen Komplexkatalysators.

Bei der Aminierungsreaktion wird erfindungsgemäß mindestens eine Hydroxylgruppe (-OH) des Edukts mit Ammoniak zu einer primären Aminogruppe (-NH₂) umgesetzt, wobei sich jeweils ein Mol Reaktionswasser pro Mol umgesetzter Hydroxylgruppe bildet.

So bilden sich bei der Umsetzung von Alkanolaminen, die nur eine Hydroxylgruppe aufweisen, die entsprechenden Diamine. Die Umsetzung von Monoaminoethanol führt somit zum entsprechenden 1,2-Diaminoethan.

Bei der Reaktion von Edukten, die eine weitere Hydroxylgruppe aufweisen (Diole), werden je nach Reaktionsbedingungen mit Ammoniak zu den entsprechenden primären Diaminen oder Alkanolaminen umgesetzt. Die Umsetzung von 1,2-Ethylenglykol führt somit zum entsprechenden 1,2-Diaminoethan oder Monoaminoethanol.

Bei der Reaktion von Edukten, die neben einer Hydroxylgruppe zwei weitere Hydroxylgruppe aufweisen (Triole), werden zwei oder drei Hydroxylgruppen mit Ammoniak zu den entsprechenden primären Diaminen oder Triaminen umgesetzt. Die Bildung von Diaminen oder Triaminen kann dabei durch die Menge des eingesetzten Ammoniaks und durch die Reaktionsbedingungen gesteuert werden. Die Umsetzung von Glycerin führt somit zum entsprechenden 1,3-Diaminopropanol (1,3-Diamin-Propan-2-ol) oder zum 1,2,3-Triaminopropan.

Bei der Reaktion von Edukten, die neben der einen Hydroxylgruppe mehr als drei weitere Hydroxylgruppe aufweisen (Polyole), werden zwei, drei oder mehr Hydroxylgruppen mit Ammoniak zu den entsprechenden primären Diaminen, Triaminen oder Polyaminen umgesetzt. Die Bildung der entsprechenden primären Diamine, Triamine oder Polyamine kann dabei durch die Menge des eingesetzten Ammoniaks und durch die Reaktionsbedingungen gesteuert werden.

Unter homogen-katalysiert wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst im flüssigen Reaktionsmedium vor (100 %), jeweils bezogen auf die Gesamtmenge im flüssigen Reaktionsmedium.

Die Menge der Metallkomponente des Katalysators, bevorzugt Ruthenium oder Iridium, beträgt im Allgemeinen 0,1 bis 5000 Gewichts-ppm, jeweils bezogen auf das gesamte flüssige Reaktionsgemisch im Reaktionsraum.

Die Umsetzung in Schritt (a) erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 110 bis 160°C durchgeführt.

Die Reaktion wird im Allgemeinen bei einem Gesamtdruck von 0.1 bis 20 MPa absolut, der sowohl der Eigendruck des Lösungsmittels und des Ammoniaks bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck bis 15 MPa absolut, insbesondere bevorzugt bei einem Gesamtdruck von bis 10 MPa absolut durchgeführt.

Das Aminierungsmittel (Ammoniak) kann bezüglich der zu aminierenden Hydroxylgruppen in stöchiometrischen, unterstöchiometrischen oder überstöchiometrischen Mengen eingesetzt werden. In einer bevorzugten Ausführungsform wird Ammoniak mit einem 1,0- bis 250-fachen, bevorzugt mit einem 1,5- bis 100-fachen, insbesondere mit einem 2- bis 10-fachen molaren Überschuss pro Mol im Edukt umzusetzender Hydroxylgruppen eingesetzt. Es sind auch höhere Überschüsse an Ammoniak möglich. Der Ammoniak kann gasförmig, flüssig oder in einem der Lösungsmittel gelöst zugegeben werden.

Erfindungsgemäß erfolgt die Umsetzung in Gegenwart mindestens eines unpolaren Lösungsmittels. Dabei kann ein unpolares Lösungsmittel oder Mischungen aus zwei oder mehr unpolaren Lösungsmitteln eingesetzt werden.

Das unpolare Lösungsmittel ist in der Regel ausgewählt aus gesättigten Kohlenwasserstoffen wie Hexan, Heptan, Oktan und Cyclohexan; linearen und zyklischen Ethern wie Diethylether, 1,4-Dioxan, tert-Butylmethylether, tert.-Amylalkohol, tert.-Butylalkohol, Diglyme und 1,2-Dimethoxyethan und aromatischen Kohlenwasserstoffen wie Benzol, Toluol, o-, m-, p-Xylol und Mesitylen sowie Mischungen davon. Bevorzugt werden aromatische Lösungsmittel, besonders bevorzugt Toluol, o-, m-, p-Xylol, Mesitylen und Mischungen davon eingesetzt.

Dabei werden unpolares Lösungsmittel und homogener Katalysator derart ausgewählt, dass sich der Katalysator in der nach der Phasentrennung in Schritt (b) erhaltenen unpolaren Phase (B) anreichert. Unter angereichert wird erfindungsgemäß verstanden, dass der Mengenverteilungskoeffizient V_{MK} = [Menge des gelösten Katalysators in der unpolaren Phase (B)] / [Menge des gelösten Katalysators in der polaren Produktphase (A)] größer 1 ist. Bevorzugt ist V_{MK} mindestens 1,5, besonders bevorzugt mindestens 5.

In einer bevorzugten Ausführungsform werden unpolares Lösungsmittel und homogener Katalysator derart ausgewählt, dass sich der Katalysator besser in der nach der Phasentrennung in Schritt (b) erhaltenen unpolaren Phase (B) löst als in der polaren Phase (A). Die Katalysatorkonzentration ist dann in der unpolaren Phase (B) höher als in der polaren Phase (A), das heißt, dass der Verteilungskoeffizient V_{K1} = [Konzentration des gelösten Katalysators in der unpolaren Phase (B)] / [Konzentration des gelösten Katalysators in der polaren Produktphase (A)] größer 1 ist. Bevorzugt ist V_{K} mindestens 1,5, besonders bevorzugt mindestens 5.

Die Auswahl des homogenen Katalysators und des unpolaren Lösungsmittels erfolgt in der Regel durch einen einfachen Versuch, bei dem der Verteilungskoeffizient V des gewählten Katalysators unter den geplanten Verfahrensbedingungen zusammen mit dem Substrat und Produkt sowie des polaren Lösungsmittel experimentell bestimmt wird. Insbesondere kann durch Wahl der Liganden die Lipophilie des Katalysators und damit seine Löslichkeit in unpolaren bzw. polaren Phasen gezielt beeinflusst werden.

In der Regel wird das unpolare Lösungsmittel so ausgewählt, dass sich der homogene Katalysator im Vergleich zum polaren Lösungsmittel bevorzugt darin löst. Dies bedeutet erfindungsgemäß, dass der Verteilungskoeffizient V_{K2} = [Konzentration des Katalysators im unpolaren Lösungsmittel] / [Konzentration des Katalysators im polaren Lösungsmittel] größer 1 ist, bevorzugt mindestens 2 und besonders bei bevorzugt mindestens 5 liegt. Weiterhin bevorzugt ist V_{K2} mindestens 1,5.

Für die Umsetzung in der Flüssigphase leitet man üblicherweise Ammoniak, den mindestens einen Alkohol, das mindestens eine unpolare Lösungsmittel zusammen mit dem Komplexkatalysator in einen Reaktionsraum. Die Reaktion kann in den üblichen, dem Fachmann bekannten Vorrichtungen bzw. Reaktoren für Flüssig-Gas-Reaktionen, bei denen der Katalysator homogen gelöst in der Flüssigphase vorliegt, durchgeführt werden. Für das erfindungsgemäße Verfahren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für gas-flüssig und für flüssig-flüssig Reaktionssysteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren. Die Zuleitung von Ammoniak, Edukt, unpolarem Lösungsmittel und Komplexkatalysator kann dabei simultan oder getrennt voneinander erfolgen. Die Reaktion kann dabei diskontinuierlich in Batchfahrweise oder kontinuierlich, semikontinuierlich mit Rückführung oder ohne Rückführung durchgeführt werden. Die mittlere Verweilzeit im Reaktionsraum beträgt im Allgemeinen 15 Minuten bis 100 Stunden.

Das in Schritt (a) enthaltene Produktgemisch (P) enthält den homogenen Katalysator, das Aminierungsprodukt, unpolares Lösungsmittel, nicht umgesetzte Edukte und ggf. gebildete Nebenprodukte sowie bei der Aminierung gebildetes Wasser. Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

In Schritt (b) des erfindungsgemäßen Verfahrens findet eine Phasentrennung des in Schritt (a) enthaltenen Produktgemisches (P) statt, ggf. nach Temperaturerniedrigung und/oder Zugabe mindestens eines polaren Lösungsmittels, das mit dem in Schritt (a) eingesetzten unpolaren Lösungsmittel eine Mischungslücke aufweist, unter Erhalt mindestens einer polaren Produktphase (A) und mindestens einer unpolaren Phase (B) enthaltend mindestens einen Teil des eingesetzten Katalysators und Abtrennen der unpolaren Phase (B).

Beide Phasen (A) und (B) sind Flüssigphasen, wobei der Katalysator in der unpolaren Phase (B) angereichert und das Aminierungsprodukt in der polaren Phase angereichert vorliegt. "Angereichert" in Hinblick auf das Aminierungsprodukt bedeutet vorliegend, dass der Mengenverteilungskoeffizient des Aminierungsprodukts V_{A} = [Menge des Aminierungsprodukts in der polaren Phase (A)] / [Menge des Aminierungsprodukts in der unpolaren Phase (B)] größer 1 ist, bevorzugt mindestens 1,5, besonders bevorzugt mindestens 5.

In einer bevorzugten Ausführungsform wird das polare Lösungsmittel derart ausgewählt, dass sich das Aminierungsprodukt besser in der nach Phasentrennung in Schritt (b) erhaltenen polaren Phase (A) löst als in der unpolaren Phase (B). Die Aminierungsproduktkonzentration ist dann in der polaren Phase (A) höher als in der unpolaren Phase (B), d.h. dass der Verteilungskoeffizient des Aminierungsprodukts V_{A1} = [Konzentration des Aminierungsprodukts in der polaren Phase (A)] / [Konzentration des Aminierungsprodukts in der unpolaren Phase (B)] größer 1 ist, bevorzugt mindestens 1,5, besonders bevorzugt mindestens 5.

Je nach Wahl der Komponenten ist es möglich, dass das Produktgemisch (P) nach Schritt (a) einphasig flüssig vorliegt. In diesem Fall kann eine Phasentrennung durch Abkühlen und/oder Zugabe eines oder mehrerer polarer Lösungsmittel erreicht werden.

Als polare Lösungsmittel eignen sich beispielsweise Wasser, Dimethylformamid, Formamid, Acetonitril, der bzw. die in Schritt (a) als Edukte eingesetzten Alkohole/Alkanolamine und Mischungen davon. Ferner kann auch das Produkt als Lösungsmittel verwendet werden. Bevorzugt wird Wasser eingesetzt. Das polare Lösungsmittel kann sowohl bereits der Reaktionsmischung vor oder in Schritt (a) als auch nach der Reaktion in Schritt (b) zusätzlich zum bei der Reaktion entstehenden Reaktionswasser zugesetzt werden.

Das polare Lösungsmittel ist erfindungsgemäß derart mit dem unpolaren Lösungsmittel und dem entstehenden Aminierungsprodukt abzustimmen, dass das Aminierungsprodukt in der polaren Phase (A) angereichert vorliegt. Die Auswahl des unpolaren und des polaren Lösungsmittels erfolgt im Allgemeinen durch einfachen Versuch, in dem die Löslichkeit des gewünschten Produkts in den beiden Phasen (A) und (B) unter den geplanten Verfahrensbedingungen experimentell bestimmt wird.

In der Regel wird das polare Lösungsmittel so gewählt, dass sich das Aminierungsprodukt im Vergleich zum eingesetzten unpolaren Lösungsmittel bevorzugt darin löst. Dies bedeutet erfindungsgemäß, dass der Verteilungskoeffizient V_{A2} = [Konzentration Aminierungsprodukt in polarem Lösungsmittel] / [Konzentration Aminierungsprodukt in unpolarem Lösungsmittel] größer 1 ist, bevorzugt mindestens 2 und besonders bevorzugt mindestens 5 ist. Weiterhin bevorzugt ist \/_{A2} mindestens 1,5.

Im Rahmen der vorliegenden Erfindung können jeweils ein Lösungsmittel oder Mischungen aus 2 oder mehr Lösungsmitteln eingesetzt werden. Dies gilt für die unpolaren als auch die polaren Lösungsmittel.

Als Maß für die Zuordnung eines Lösungsmittels zur Gruppe polar/unpolar kann die Dielektrizitätskonstante DK herangezogen werden. Lösungsmittel mit DK größer als etwa 15 werden üblicherweise als polar angesehen (z. B. weist Acetonitril ein DK von etwa 37 auf), Lösungsmittel mit niedrigerer DK werden üblicherweise als unpolar angesehen, beispielsweise beträgt die DK von Benzol 2,28.

Auch falls das in Schritt (a) enthaltene Produktgemisch bereits zweiphasig vorliegt, kann die Zugabe von polarem Lösungsmittel vorteilhaft sein, wenn dadurch eine günstigere Verteilung des Katalysators und des Aminierungsprodukts auf die beiden Phasen (A) und (B) erzielt wird. Gleiches gilt für die Temperaturerniedrigung.

Die Phasentrennung der beiden Phasen (A) und (B) in Schritt (b) erfolgt im Allgemeinen durch gravimetrische Phasentrennung. Als Phasentrenngefäß kann der Reaktionsraum dienen, in dem die Umsetzung gemäß Schritt (a) stattgefunden hat, beispielsweis ein Reaktor. Die Trennung von zwei Flüssigphasen ist an sich eine Standardprozedur, die dem Fachmann bekannt ist. Standardmethoden und -verfahren sind beispielsweise in E. Müller et al., "Liquid-Liquid Extraction" in Ullmann's Encyclopedia of Industrial Chemistry, 2005, Wiky-VCH-Verlag, 10.1002/14356007.603-06, Kapitel 3, "Apparatus", beschrieben.

In Schritt (c) des erfindungsgemäßen Verfahrens wird mindestens ein Teil der in Schritt (b) abgetrennten Phase (B), ggf. nach einem oder mehreren Schritten zur Aufreinigung wie Destillation wieder in die Umsetzung in Schritt (a) rückgeführt. Dabei kann die gesamte abgetrennte unpolare Phase (B) rückgeführt werden, es kann jedoch auch sinnvoll sein, zur Ausschleusung von unerwünschten Nebenprodukten und Verunreinigungen einen Teil der unpolaren Phase aus dem Verfahren auszuschleusen, um eine Anreicherung unerwünschter Komponenten im Verfahren zu vermeiden.

In der Regel wird auch die polare Phase zumindest geringe Mengen des Katalysators enthalten. Falls es nötig ist, den Katalysatoranteil in der polaren Phase (A) weiter abzusenken, so kann diese mit unpolarem Lösungsmittel extrahiert werden. Die Extraktion des Katalysators kann in jeder geeigneten, dem Fachmann bekannten Vorrichtung durchgeführt werden, bevorzugt in Gegenstrom-Extraktionskolonnen, Mixer-Settler-Kaskaden oder Kombinationen von Mixer-Settler-Apparaturen mit Extraktionskolonnen. Das den Katalysator enthaltende, unpolare Extrakt kann dann ggf. nach Entfernung überschüssigen unpolaren Lösungsmittels durch Einengen wieder in die Aminierungsreaktion in Schritt (a) zurückgeführt werden. Vorzugsweise wird als Extraktionsmittel das in Schritt (a) eingesetzte unpolare Lösungsmittel verwendet. Die Extraktion des Katalysators aus der polaren Produktphase (B) kann vor oder nach der Abtrennung des Aminierungsprodukts in Schritt (d) durchgeführt werden. Gemäß einer bevorzugten Ausführungsform wird der extrahierte Katalysator zumindest teilweise in die Umsetzung rückgeführt.

In Schritt (d) des erfindungsgemäßen Verfahrens wird das Aminierungsprodukt aus der polaren Produktphase (A) abgetrennt. So kann in Schritt (d) das polare Lösungsmittel durch Destillation vom Aminierungsprodukt abgetrennt werden und entweder in das Verfahren rückgeführt werden oder verworfen werden. Nicht umgesetztes Edukt (Alkohol), gegebenenfalls vorhandene überschüssiger Ammoniak oder Nebenprodukte können ebenfalls destillativ vom Aminierungsprodukt entfernt werden. Die thermische Abtrennung des polaren Lösungsmittels erfolgt nach den dem Fachmann bekannten Verfahren des Standes der Technik, bevorzugt in einem Verdampfer oder in einer Destillationseinheit, umfassend Verdampfer und Kolonne(n), die üblicherweise mehrere Böden, eine Packung oder Füllkörper aufweist.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, die in einer Menge von 0,01 bis 100 molaren Äquivalenten in Bezug auf den verwendeten Metallkatalysator eingesetzt werden können.

Die Erfindung wird durch die nachfolgenden Beispiele verdeutlicht, ohne sie darauf zu beschränken.

### Beispiele:

Generelle Vorschrift für die erfindungsgemäße katalytische Aminierung von Alkoholen mit Ammoniak Ligand L, Metallsalz M oder Katalysatorkomplex XIVb (Herstellung, siehe unten, Einwaage unter interter Atmosphäre), Lösungsmittel (so viel, dass Gesamtlösungsmittelvolumen 50 ml beträgt) und der umzusetzende Alkohol wurden unter Ar-Atmosphäre in einem 160 ml Parr-Autoklaven (Edelstahl V4A) mit magnetisch gekoppeltem Schrägblattrührer (Rührgeschwindigkeit: 200-500 Umdrehungen/Minute) vorgelegt. Die angegebene Menge an Ammoniak wurde bei Raumtemperatur entweder vorkondensiert oder direkt aus der NH₃-Druckgasflasche zudosiert. Falls Wasserstoff eingesetzt wurde, erfolgte dies durch iterative Differenzdruck-Dosierung. Der Stahlautoklav wurde auf die angegebene Temperatur elektrisch aufgeheizt und für die angegebene Zeit unter Rühren (500 Umdrehungen/Minute) erhitzt (Innentemperaturmessung). Nach dem Abkühlen auf Raumtemperatur, Entspannung des Autoklaven und Ausgasen des Ammoniaks bei Normaldruck wurden 5 oder 50 mL Wasser zugegeben, wobei zwei Flüssigphasen erhalten wurden, die durch Phasenseparation voneinander getrennt wurden. Die Reaktionsmischung wurde mittels GC (30m RTX5 Amin 0,32mm 1,5µm) analysiert. Der Rutheniumgehalt der jeweiligen Flüssigphase wurde durch Atomadsorptionsspektroskopie ermittelt. Die Ergebnisse für die Aminierung von 1,4-Butandiol (Tabelle 1), Diethylenglykol (Tabelle 2) und Monoethylenglykol (Tabelle 3) sind im Folgenden angegeben:

### Synthese des Katalysatorkomplexes XIVb

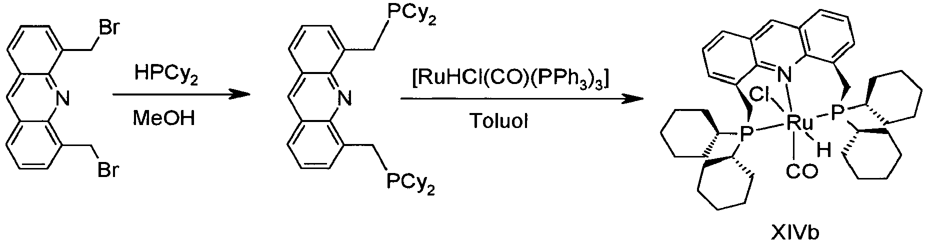

### a) Synthese von 4,5-Bis(dicyclohexylphosphinomethyl)acridin

Eine Lösung aus 4,5-Bis(bromomethyl)acridin¹ (5,2 g, 14,2 mmol) und Dicyclohexylphosphin (8,18 g, 36,8 mmol) in 65 mL wasserfreiem, entgasten Methanol wurde 66h auf 50°C unter einer inerten Argon-Atmosphäre erhitzt. Nach Abkühlen auf Raumtemperatur wurde Triethylamin (5,72 g, 56,7 mmol) zugegeben und 1 h gerührt. Verdampfen des Lösungsmittels ergab einen gelb-weißen Feststoff in rotem Öl. Extraktion mittels 3 x 40 mL MTBE und Konzentration des Filtrats ergaben ein rotbraunes Öl (¹H NMR: Mischung aus Produkt & HPCy₂). Aufnehmen in wenig warmen MTBE gefolgt von Zugabe von eisgekühltem Methanol resultierte in Ausfällung eines gelben, mikrokristallinen Feststoffes. Isolierung und Trocknung im Vakuum ergab luftempfindliches 4,5-Bis(dicyclohexylphosphinomethyl)acridin (2,74 g, 33%) als gelbes Pulver. ¹H NMR (360,63 MHz, Toluol-d8): δ [ppm] = 8,07 (s, 1 H, H9), 7,91 (d, *J* = 8,3 Hz, 2H, Ar-H), 7,42 (d, *J* = 8,3 Hz, 2H, Ar-H), 7,21 (dd, *J* = 8,3 Hz, *J* = 7,2 Hz, 2H, Ar-H), 3,89 (bs, 4H, -C*H*₂-P), 1,96-1,85 (m, 8H, Cy-H), 1,77-1,54 (m, 20H, Cy-H), 1,26-1,07 (m, 16H, Cy-H). ³¹P{¹H} NMR (145,98 MHz, Toluol-d8): δ [ppm] = 2,49 (s, -CH₂-*P*(Cy)₂).

### b) Synthese des Katalysatorkomplexes XIVb

4,5-Bis(dicyclohexylphosphinomethyl)acridin (1855 mg, 3,1 mmol) und [RuHCl(CO)(PPh₃)₃]² (2678 mg, 2,81 mmol) wurden für 2h in 80 mL entgastem Toluol auf 70°C erhitzt. Die resultierende dunkelbraune Lösung wurde zur Trockene eingeengt, der Rückstand in 3 x 20 mL Hexan aufgeschlämmt und durch Filtration isoliert. Trocknen im Vakuum ergab Ru-PNP-Pincer-Komplex XIVb (1603 mg, 75%) als orange-braunes Pulver. ¹H NMR (360,63 MHz, Toluol-d8): δ [ppm] = 8,06 (s, 1H, H9), 7,43 (d, *J* = 7,6 Hz, 2H, Ar-H), 7,33 (d, *J* = 6,5 Hz, 2H, Ar-H), 7,06-7,02 (m, 2H, Ar-H), 5,02 (d, *J* = 11,9 Hz, 2H, -CHH-PCy₂), 3,54 (d, *J* = 12,2 Hz, 2H, -CHH-PCy₂), 2,87 (bs, 2H, - P(CₐH(CH₂)₅)₂), 2,54 (bs, 2H, -P(C_{b}*H*(CH₂)₅)₂), 2,18 (bs, 2H, Cy-H), 1,88-1,85 (m, 8H, Cy-H), 1,65 (bs, 6H, Cy-H), 1,42-1,35 (m, 14H, Cy-H), 1,17-0,82 (m, 12H, Cy-H), - 16,29 (t, *J* = 19,1 Hz, 1H, Ru-H). ³¹P{¹H} NMR (145,98 MHz, Toluol-d8): δ [ppm] = 60,89 (s, -CH₂-*P*(Cy)₂).
¹ J. Chiron, J.P. Galy, Synlett, 2003, 15, 2349-2350.
² Literaturvorschrift: Inorganic Syntheses 1974, 15, 48. Siehe auch: T. Joseph, S. S. Deshpande, S. B. Halligudi, A. Vinu, S. Ernst, M. Hartmann, J. Mol. Cat. (A) 2003, 206, 13-21.

**Tabelle 1: Umsetzung von 1,4-Butandiol**

| | | | | | | | | | | | | | | |
|---|---|---|---|---|---|---|---|---|---|---|---|---|---|---|
| 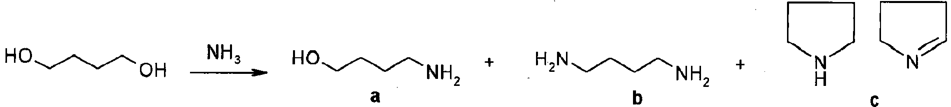 | | | | | | | | | | | | | | |

| **Nr^{a)}** | **T (°C)** | **Zeit (h)** | **NH₃ [Eq.] ^{e)}** | **Reaktionsdruck [bar]** | **Metallsalz** | **Metall [M] (mol%)^{r)}** | **Ligand [L] (mol%)** | **Ligand [L] (mol%) ^{r)}** | **V_{mK} (Ru) ^{q)}** | **V_{K} (Ru) q)** | **Umsatz ^{b)}** | **Selektivität^{c)}** | | |
|---|---|---|---|---|---|---|---|---|---|---|---|---|---|---|
| | | | | | | | | | | | | **a :** | **b :** | **c** |
| 1^{d)} | 155 | 12 | 6 | 49 | [RuHCl(CO)(PPh₃)₃] | 0,1 | Triphos¹⁾ | 0,1 | 1,8 | | 74,7 | 59,1 | 0,7 | 6,7 |
| 2^{d)} | 155 | 12 | 6 | 66^{h)} | [RuHCl(CO)(PPh₃)₃] | 0,1 | Triphos^{f)} | 0,1 | 3,2 | | 61,8 | 78,0 | 0,6 | 5,4 |
| 3^{d)} | 155 | 12 | 6 | 45 | [RuHCl(CO)(PPh₃)₃] | 0,1 | Xantphos^{g)} | 0,1 | 1,4 | | 35,0 | 81,8 | 0,0 | 6,4 |
| 4^{d)} | 155 | 60 | 6 | 61^{h)} | [RuHCl(CO)(PPh₃)₃] | 0,2 | Triphos^{f)} | 0,2 | 11,5 | 1,7 | 99,5 | 23,6 | 8,2 | 62,3 |
| 5^{d)} | 155 | 60 | 6 | 61^{h)} | [RuHCl(CO)(PPh₃)₃] | 0,1 | Triphos^{f)} | 0,1 | 14,0 | 2,0 | 98,5 | 34,2 | 11,4 | 49,6 |
| 6^{d)} | 155 | 12 | 6 | 81ⁱ⁾ | [RuHCl(CO)(PPh₃)₃] | 0,1 | Triphos^{f)} | 0,1 | 10,0 | 1,4 | 10,5 | 97,3 | 0 | 0,9 |
| 7^{d)} | 180 | 12 | 6 | 89^{j)} | [RuHCl(CO)(PPh₃)₃] | 0,1 | Triphos^{f)} | 0,1 | 12,0 | 1,7 | 9,2 | 97,5 | 0 | 0,8 |
| 8^{k)} | 180 | 12 | 6 | 90ⁱ⁾ | Katalysatorkomplex XIVb | 0,1 | - | | 3,5 | 3,6 | 74,8 | 44,7 | 2,1 | 41,0 |
| g^{d)} | 155 | 12 | 6 | 41 | [RuHCl(CO)(PPh₃)₃] | 0,1 | Triphos^{f)} | 0,1 | 2,1 | | 66,3 | 62,1 | 0,5 | 5,8 |
| 10^{d)} | 155 | 12 | 6 | 41 | [RuHCl(CO)(PPh₃)₃] | 0,1 | Xantphos^{g)} | 0,1 | 1,6 | | 34,3 | 79,6 | 0 | 4,9 |
| 11^{d)} | 155 | 12 | 6 | 41 | Katalysatorkomplex XIVb | 0,1 | | | 1,1 | | 63,0 | 71,8 | 9,3 | 17,3 |
| 12^{d)} | 155 | 12 | 6 | 61^{h)} | [RuHCl(CO)(PPh₃)₃] | 0,1 | Xanthphos^{g)} | 0,1 | 1,4 | | 29,3 | 79,8 | 0 | 3,3 |
| 13^{d)} | 155 | 12 | 6 | 61^{h)} | [Ru(COD)methylallyl2 ] | 0,1 | (Tetraphos)^{l)} | 0,1 | 1,3 | | 10,9 | 33,2 | 0 | 0,6 |
| 14^{d)} | 155 | 12 | 6 | 55^{h)} | Katalysatorkomplex XIVb | 0,1 | | | 1,5 | | 25 | 81 | 4,8 | 13,6 |
| 15^{d)} | 180 | 12 | 6 | 39^{h)} | [RuHCl(CO)(PPh₃)₃] | 0,2 | Triphos^{f)} | 0,2 | 5,7 | | 99,9 | 1,7 | 4,7 | 37,7 |
| 16^{d)} | 155 | 12 | 6 | 35 | [RuHCl(CO)(PPh₃)₃] | 0,2 | Triphos^{f)} | 0,2 | 1,8 | | 75,5 | 60,4 | 0,7 | 20,5 |
| 17^{d)} | 155 | 12 | 6 | 40 | Katalysatorkomplex XIVa | 0,1 | | | 1,2 | | 56,1 | 79,7 | 3 | 16,3 |
| 18^{d)} | 180 | 2 | 6 | 53 | Katalysatorkomplex XIVb | 0,2 | | | 1,1 | | 91,5 | 36,4 | 25,8 | 35,4 |
| 19^{d)} | 180 | 12 | 6 | 70^{h)} | Katalysatorkomplex XIVb | 0,1 | | | 1,2 | | 94,2 | 30,1 | 37,2 | 31,6 |
| 20^{d)} | 155 | 12 | 1,5 | 11 | Katalysatorkomplex XIVb | 0,1 | | | 1,1 | | 81,6 | 35,4 | 9 | 51 |
| 21^{d)} | 155 | 12 | 6 | 70ⁿ⁾ | [RuHCl(CO)(PPh₃)₃] | 0,1 | Triphos^{f)} | 0,1 | 3,4 | | 19,8 | 95,4 | 0 | 1,5 |
| 22^{d)} | 155 | 12 | 6 | 38 | [RuHCl(CO)(PPh₃)₃] | 0,2 | DPPEPP^{p)} | 0,2 | 1,0 | | 66,6 | 68,1 | 0,1 | 11 |
| 23^{d)o)} | 155 | 12 | 6 | 41 | [RuHCl(CO)(PPh₃)₃] | 0,2 | DPPEPP^{p)} | 0,2 | 4,3 | | 39,4 | 56,2 | 0 | 4,3 |
| 24^{d)} | 180 | 12 | 6 | 82ⁿ⁾ | Katalysatorkomplex XIVb | 0,1 | | | 2,3 | | 89,9 | 36,2 | 35,4 | 27,7 |
| a) 50ml Toluol; Ansatzgröße: 25 mmol 1,4-Butandiol, | | | | | | | | | | | | | | |
| b) Auswertung per GC (FI.%); | | | | | | | | | | | | | | |
| c) Produktselektivität bestimmt per GC; | | | | | | | | | | | | | | |
| d) Ru-Verteilungskoeffizient wurde anhand der gemessen Ru-Gehalte in 50 ml organischem Lösungsmittel und 5 ml Wasser (Zusatz nach Reaktionsende) bestimmt; | | | | | | | | | | | | | | |
| e) Molare Equivalente NH₃ pro OH-Funktion am Substrat; | | | | | | | | | | | | | | |
| f) Triphos = 1,1,1-Tris(diphenylphosphino-Methyl)ethan, CAS 22031-12-5; | | | | | | | | | | | | | | |
| g) Xantphos = 4,5-Bis(diphenylphosphino)-9,9-dimethylxanthen, CAS 161265-03-8; | | | | | | | | | | | | | | |
| h) 5 bar H₂ kalt aufgepresst; | | | | | | | | | | | | | | |
| i) 20 bar H₂ kalt aufgepresst; | | | | | | | | | | | | | | |
| j) 30 bar H₂ kalt aufgepresst; | | | | | | | | | | | | | | |
| k) Ru-Verteilungskoeffizient wurde anhand der gemessen Ru-Gehalte in 50 ml organischem Lösungsmittel und 50 ml Wasser (Zusatz nach Reaktionsende) bestimmt, | | | | | | | | | | | | | | |
| l) Tetraphos = Tris[2-(diphenylphosphino)ethyl]phosphin, CAS 23582-03-8, | | | | | | | | | | | | | | |
| m) 5 mol% Wasser pro OH-Funktion am Substrat, | | | | | | | | | | | | | | |
| n) 10 bar H₂ kalt aufgepresst, | | | | | | | | | | | | | | |
| o) 0,2 mol% Kalium-tert-butanolat, | | | | | | | | | | | | | | |
| p) Bis(2-diphenylphosphino-ethyl)phenylphosphin, CAS: 23582-02-7 | | | | | | | | | | | | | | |
| q) VmK = mRu (Oberphase) / mRu (Unterphase); VK = cRu (Oberphase) / cRu /Unterphase); | | | | | | | | | | | | | | |
| r) mol% bezogen auf Anzahl der OH-Funktionen am Substrat | | | | | | | | | | | | | | |

**Tabelle 2: Umsetzung von Diethylenglykol**

| | | | | | | | | | | | | | |
|---|---|---|---|---|---|---|---|---|---|---|---|---|---|
| 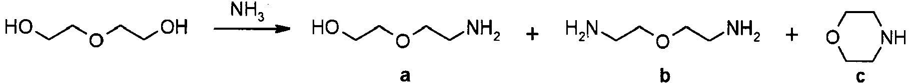 | | | | | | | | | | | | | |

| **Nr^{a)}** | **T(°C)** | **Zeit (h)** | **Reakti onsdruck [bar]** | **Metallsalz** | **Metall [M] (mol% )^{h)}** | **Ligand [L]** | **Lig. (mol%)^{h)}** | **V_{K} (m) (Ru)^{e)}** | **V_{K}(c) (Ru)^{e)}** | **Umsatz ^{b)}** | **Selektivität ^{c}** | | |
|---|---|---|---|---|---|---|---|---|---|---|---|---|---|
| | | | | | | | | | | | **a :** | **b :** | **c** |
| 1^{d)} | 155 | 12 | 59^{k)} | [RuHCl(CO)(PPh₃)₃] | 0,1 | Triphos^{f)} | 0,1 | 2,5 | | 16,2 | 87,3 | 0,1 | 2,3 |
| 2^{d)} | 135 | 12 | 37 | Katalysatorkomplex XIVb | 0,1 | | | 3,3 | | 42,1 | 87,1 | 3,3 | 6,5 |
| 3^{d)} | 155 | 12 | 43 | Katalysatorkomplex XIVb | 0,1 | | | 1,7 | | 82,4 | 55,3 | 20,1 | 10,9 |
| 4^{d)} | 180 | 12 | 87^{m)} | [RuHCl(CO)(PPh₃)₃] | 0,2 | Triphos^{f)} | 0,2 | 15,6 | 2,3 | 24,1 | 93,0 | 1,2 | 5,3 |
| 5^{g)} | 155 | 12 | 67^{l)} | Katalysatorkomplex XIVb | 0,1 | | | 1,9 | 2,0 | 12,2 | 94,1 | 2,1 | 2,0 |
| 6^{g)} | 155 | 12 | 82^{m)} | Katalysatorkomplex XIVb | 0,1 | | | 5,9 | 6,1 | 4,2 | 93,3 | 0 | 2,1 |
| 7^{g)} | 155 | 12 | 94ⁿ⁾ | Katalysatorkomplex XIVb | 0,1 | | | 5,4 | 5,7 | 2,2 | 91,9 | 0 | 0 |
| 8^{g)} | 155 | 12 | 43 | Katalysatorkomplex XIVb | 0,1 | | | 1,9 | 2,0 | 79,6 | 57,6 | 22,4 | 12,8 |
| 9^{d)} | 155 | 12 | 42,6 | [RuHCl(CO)(PPh₃)₃] | 0,10 | Xanthphosⁱ⁾ | 0,10 | 1,1 | | 27,7 | 67,1 | 0,2 | 5,26 |
| 10^{d) o)} | 155 | 15 | 46,4 | Katalysatorkomplex XIVb | 0,10 | | | 1,1 | | 77,5 | 49,1 | 23,7 | 13,08 |
| 11^{d)} | 155 | 12 | 41,8 | [RuHCl(CO)(PPh₃)₃] | 0,10 | Triphos^{f)} | 0,10 | 1,0 | | 52,4 | 66,2 | 0,9 | 6,60 |
| 12^{d)} | 155 | 12 | 61,2^{k)} | [RuHCl(CO)(PPh₃)₃] | 0,10 | Xanthphosⁱ⁾ | 0,10 | 1,2 | | 19,0 | 75,0 | 0,1 | 5,85 |
| 13^{d)} | 155 | 12 | 57,1^{k)} | Katalysatorkomplex XIVb | 0,10 | | | 1,3 | | 29,4 | 87,0 | 6,7 | 3,58 |
| 14^{d)} | 180 | 12 | 64,7 | [RuHCl(CO)(PPh₃)₃] | 0,20 | Triphos^{f)} | 0,20 | 2,0 | | 97,6 | 26,4 | 13,4 | 53,98 |
| ^{15d) q)} | 155 | 12 | 7,5 | Katalysatorkomplex XIVb | 0,10 | | | 1,4 | | 85,9 | 18,4 | 7,5 | 41,55 |
| 16^{d)} | 155 | 12 | 58,8 | [RuHCl(CO)(PPh₃)₃] | 0,40 | Triphos^{f)} | 0,40 | 1,1 | | 64,7 | 65,2 | 1,3 | 17,97 |
| 17^{d)} | 180 | 12 | 61,1^{k)} | [RuHCl(CO)(PPh₃)₃] | 0,20 | Triphos^{f)} | 0,20 | 1,7 | | 93,0 | 43,7 | 11,6 | 38,76 |
| 18^{d)} | 180 | 12 | 60,6^{k)} | [RuHCl(CO)(PPh₃)₃] | 0,40 | Triphos^{f)} | 0,40 | 2,4 | | 95,3 | 38,0 | 9,8 | 48,00 |
| 19^{d)} | 155 | 60 | 60,1^{k)} | [RuHCl(CO)(PPh₃)₃] | 0,20 | Triphos^{f)} | 0,20 | 6,0 | | 86,1 | 52,4 | 6,9 | 35,35 |
| 20^{d)} | 155 | 60 | 58,8^{k)} | [RuHCl(CO)(PPh₃)₃] | 0,10 | Triphos^{f)} | 0,10 | 3,4 | | 85,1 | 52,8 | 8,3 | 31,65 |
| 21^{d) p)} | 155 | 12 | 42,2 | [RuHCl(CO)(PPh₃)₃] | 0,20 | DPPEPP^{J)} | 0,20 | 2,0 | | 23,1 | 46,9 | 0,0 | 5,51 |
| 22^{d)} | 180 | 12 | 75,6^{l)} | Katalysatorkomplex XIVb | 0,10 | | | 2,3 | | 86,1 | 35,7 | 47,2 | 13,77 |
| 23^{d)} | 180 | 12 | 95,9^{m)} | Katalysatorkomplex XIVb | 0,10 | | | 3,4 | | 29,1 | 71,8 | 18,2 | 5,32 |
| 24^{d)} | 180 | 12 | 79,7^{l)} | [RuHCl(CO)(PPh₃)₃] | 0,20 | Triphos^{f)} | 0,20 | 6,4 | | 54,1 | 83,5 | 3,3 | 11,96 |
| | a) 50ml Toluol; Ansatzgröße: 25 mmol Diethylenglykol, 6 molare Equivalente NH3 pro OH-Funktion am Substrat (wenn nicht anders angegeben) | | | | | | | | | | | | |
| | b) Auswertung per GC (FI.%); | | | | | | | | | | | | |
| | c) Produktselektivität bestimmt per GC; | | | | | | | | | | | | |
| | d) Ru-Verteilungskoeffizient wurde anhand der gemessen Ru-Gehalte in 50 ml organischem Lösungsmittel und 5 ml Wasser (Zusatz nach Reaktionsende) bestimmt; | | | | | | | | | | | | |
| | e) VmK = mRu (Oberphase) / mRu (Unterphase); VK = cRu (Oberphase) / cRu /Unterphase); | | | | | | | | | | | | |
| | f) Triphos = 1,1,1-Tris(diphenylphosphino-Methyl)ethan, CAS 22031-12-5; | | | | | | | | | | | | |
| | g) Ru-Verteilungskoeffizient wurde anhand der gemessen Ru-Gehalte in 50 ml organischem Lösungsmittel und 50 ml Wasser (Zusatz nach Reaktionsende) bestimmt, | | | | | | | | | | | | |
| | h) mol% bezogen auf Anzahl der OH-Funktionen am Substrat, | | | | | | | | | | | | |
| | i) Xantphos = 4,5-Bis(diphenylphosphino)-9,9-dimethylxanthen, CAS 161265-03-8; | | | | | | | | | | | | |
| | j) Bis(2-diphenylphosphino-ethyl)phenylphosphin, CAS: 23582-02-7 | | | | | | | | | | | | |
| | k) 5 bar H₂ kalt aufgepresst; | | | | | | | | | | | | |
| | l) 10 bar H₂ kalt aufgepresst | | | | | | | | | | | | |
| | m) 20 bar H₂ kalt aufgepresst; | | | | | | | | | | | | |
| | n) 30 bar H₂ kalt aufgepresst; | | | | | | | | | | | | |
| | o) 5 mol% Wasser pro OH-Funktion am Substrat, | | | | | | | | | | | | |
| | p) 0,2 mol% Kalium-tert-butanolat, | | | | | | | | | | | | |
| | q) nur 1 molares Equivalent NH₃ pro OH-Funktion am Substrat | | | | | | | | | | | | |

**Tabelle 3: Umsetzung von MEG**

| | | | | | | | | | | | | | | |
|---|---|---|---|---|---|---|---|---|---|---|---|---|---|---|
| 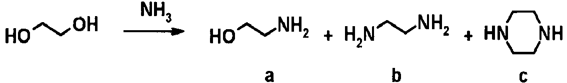 | | | | | | | | | | | | | | |

| **Nr^{a)}** | **T(°C)** | **Zeit (h)** | **Reaktionsdruck [bar]** | **Weitere Bedingungen ^{g)}** | **Metallsalz** | **Met.all [M] (mol%)^{g)}** | **Ligand [L]^{h)}** | **Lig. (mol%)^{g)}** | **V_{K} (m) (Ru)^{d,e)}** | **V_{K}(c) (Ru)^{d,,f)}** | **Umsatz^{b}** | **Selektivität ^{c}** | | |
|---|---|---|---|---|---|---|---|---|---|---|---|---|---|---|
| | | | | | | | | | | | | **a :** | **b** | **: c** |
| 1 | 180 | 12 | 36 | | [RuHCl(CO)(PPh₃)₃] | 0,20 | Triphos | 0,20 | 20,4 | 2,6 | 45,2 | 8,8 | 1,5 | 59,9 |
| 2 | 155 | 12 | 42 | | [RuHCl(CO)(PPh₃)₃] | 0,20 | Triphos | 0,20 | 13,3 | 1,7 | 20,8 | 23,2 | 3,6 | 40,4 |
| 3 | 155 | 12 | 57 | | [RuHCl(CO)(PPh₃)₃] | 0,20 | Triphos | 0,20 | 21,0 | 2,7 | 19,9 | 25,1 | 3,4 | 34,4 |
| 4 | 155 | 12 | 57 | | [RuHCl(CO)(PPh₃)₃] | 0,40 | Triphos | 0,40 | 14,4 | 1,8 | 34,3 | 19,9 | 2,8 | 15,3 |
| 5 | 180 | 12 | 48 | 1mol% KOtBu | [RuHCl(CO)(PPh₃)₃] | 0,20 | Triphos | 0,20 | 19,4 | 2,5 | 43,8 | 34,0 | 13,0 | 20,6 |
| 6 | 155 | 12 | 42 | 1mol% KOtBu | [RuHCl(CO)(PPh₃)₃] | 0,20 | Triphos | 0,20 | 9,3 | 1,2 | 20,0 | 50,5 | 11,1 | 6,9 |
| 7 | 180 | 12 | 47 | 5bar H₂ kalt aufpressen | [RuHCl(CO)(PPh₃)₃] | 0,20 | Triphos | 0,20 | 8,0 | 1,0 | 41,2 | 10,3 | 2,8 | 57,0 |
| 8 | 155 | 12 | 42 | 5bar H₂ kalt aufpressen | [RuHCl(CO)(PPh₃)₃] | 0,20 | Triphos | 0,20 | 19,9 | 2,6 | 21,6 | 22,2 | 3,5 | 33,9 |
| 9 | 180 | 12 | 51 | 5mol% H₂O | [RuHCl(CO)(PPh₃)₃] | 0,20 | Triphos | 0,20 | 11,9 | 1,5 | 39,3 | 11,1 | 3,01 | 54,6 |
| 10 | 155 | 12 | 40 | 5mol% H₂O | [RuHCl(CO)(PPh₃)₃] | 0,20 | Triphos | 0,20 | 17,7 | 2,3 | 19,6 | 21,7 | 3,1 | 33,5 |
| 11 | 155 | 60 | 57 | 5bar H₂ kalt aufpressen | [RuHCl(CO)(PPh₃)₃] | 0,20 | Triphos | 0,20 | 63,9 | 8,2 | 57,6 | 14,0 | 5,4 | 62,1 |
| 12 | 155 | 60 | 57 | 5bar H₂ kalt aufpressen | [RuHCl(CO)(PPh₃)₃] | 0,10 | Triphos | 0,10 | 24,9 | 3,2 | 35,0 | 15,7 | 7,1 | 63,6 |
| a) 50ml Toluol; Ansatzgröße: 25 mmol Ethylenglykol, 6 molare Equivalente NH₃ pro OH-Funktion am Substrat; | | | | | | | | | | | | | | |
| b) Auswertung per GC (Fl.%); | | | | | | | | | | | | | | |
| c) Produktselektivität bestimmt per GC; | | | | | | | | | | | | | | |
| d) Ru-Verteilungskoeffizient wurde anhand der gemessen Ru-Gehalte in 50 ml organischem Lösungsmittel und 5 ml Wasser bestimmt; | | | | | | | | | | | | | | |
| e) V_{K} (m) = m_{Ru} (Oberphase) / m_{Ru} (Unterphase); | | | | | | | | | | | | | | |
| f) V_{K} (c) = C_{Ru} (Oberphase) / c_{Ru} (Unterphase); | | | | | | | | | | | | | | |
| g) mol% bezogen auf Anzahl der OH-Funktionen am Substrat | | | | | | | | | | | | | | |
| h) Triphos = 1,1,1-Tris(diphenylphosphino-Methyl)ethan, CAS 22031-12-5 | | | | | | | | | | | | | | |
| ; | | | | | | | | | | | | | | |

## Patentansprüche

1. Verfahren zur Herstellung von primären Aminen durch Alkoholaminierung von Alkoholen mit Ammoniak unter Wasserabspaltung umfassend die Schritte
(a) homogen-katalysiertes Umsetzen eines Reaktionsgemischs enthaltend mindestens einen Alkohol, Ammoniak, mindestens ein unpolares Lösungsmittel und mindestens einen Katalysator enthaltend mindestens ein Element ausgewählt aus den Gruppen 8, 9 und 10 des Periodensystems in der Flüssigphase, wobei ein Produktgemisch (P) erhalten wird,
(b) Phasentrennung des in Schritt (a) erhaltenen Produktgemischs (P) ggf. nach Temperaturemiedrigung, Druckerniedrigung und/oder Zugabe mindestens eines polaren Lösungsmittels, das mit dem unpolaren Lösungsmittel eine Mischungslücke aufweist, unter Erhalt mindestens einer polaren Produktphase (A) und mindestens einer unpolaren Phase (B) enthaltend mindestens einen Teil des eingesetzten Katalysators und Abtrennen der unpolaren Phase (B),
(c) Rückführen mindestens eines Teils der unpolaren Phase (B) in die Umsetzung in Schritt (a) und
(d) Abtrennen des Aminierungsprodukts aus der polaren Produktphase (A),
wobei das in (a) eingesetzte unpolare Lösungsmittel und der in Schritt (a) eingesetzte Katalysator so gewählt werden, dass sich der Katalysator In der unpolaren Phase (B) anreichert, wobei die Alkohole mindestens eine primäre oder sekundäre Alkoholgruppe und mindestens eine zwelte funktionelle Gruppe ausgewählt aus primärer oder sekundärer Alkoholgruppe und primärer Aminogruppe aufweisen.

2. Verfahren nach Anspruch 1, **dadurch gekennzeichnet, dass** es sich bei den primaren Aminen um Alkanolamine, Diamine, Triamine oder Polyamine handelt.

3. Verfahren nach Anspruch 1 oder 2, **dadurch gekennzeichnet, dass** die Alkohole mindestens eine primäre oder sekundäre Alkoholgruppe aufweisen.

4. Verfahren nach einem der Ansprüche 1 bis 3, **dadurch gekennzeichnet, dass** in Schritt (a) und/oder (b) mindestens ein polares Lösungsmittel eingesetzt wird, das mit dem unpolaren Lösungsmittel eine Mischungslücke aufweist, und in dem sich das Aminierungsprodukt besser löst als In dem unpolaren Lösungsmittel.

5. Verfahren nach einem der Ansprüche 1 bis 4, **dadurch gekennzeichnet, dass** der Mengenverteilungskoeffizient V_{MX} = [Menge des gelösten Katalysators in der unpolaren Phase (B)] / [Menge des gelösten Katalysators in der polaren Produktphase (A)] mindestens 1,5 ist

6. Verfahren nach einem der Ansprüche 1 bis 5, **dadurch gekennzeichnet, dass** der Mengenverteilungskoeffizient V_{MA} = [Menge des Aminierungsprodukts in der polaren Phase (A)] / [Menge des Aminierungsprodukts in der unpolaren Phase (B)] mindestens 1,5 ist.

7. Verfahren nach einem der Ansprüche 1 bis 6, **dadurch gekennzeichnet, dass** das mindestens eine polare Lösungsmittel ausgewählt ist aus Wasser, Dimethylformamid, Formamid, Acetonitril, dem in Schritt (a) eingesetzten Alkohol und Mischungen davon.

8. Verfahren nach einem der Ansprüche 1 bis 7, **dadurch gekennzeichnet, dass** das unpolare Lösungsmittel ausgewählt ist aus gesättigten Kohlenwasserstoffen wie Hexan, Heptan, Oktan und Cyclohexan; linearen und zyklischen Ethem wie Diethylether, 1,4-Dioxan, tert-Butylmethylether, Diglyme und 1,2.Dimethoxyethan und aromatischen Kohlenwasserstoffen wie Benzol, Toluol, o-, m-, p-Xylol und Mesitylen sowie Mischungen davon.

9. Verfahren nach einem der Ansprüche 1 bis 8, **dadurch gekennzeichnet, dass** vorder Abtrennung des Aminierungsprodukts in Schritt (d) der in der polaren Produktphase (B) enthaltene Katalysator mit dem in Schritt (a) eingesetzten unpolaren Lösungsmittel extrahiert wird.

10. Verfahren nach Anspruch 9, **dadurch gekennzeichnet, dass** der extrahierte Katalysator zumindest teilweise in die Umsetzung rückgeführt wird.

11. Verfahren nach einem der Ansprüche 1 bis 10, **dadurch gekennzeichnet, dass** in Schritt (d) das Aminierungsprodukt vom polaren Lösungsmittel durch Destillation abgetrennt wird.

12. Verfahren nach einem der Ansprüche 1 bis 11, **dadurch gekennzeichnet, dass** der Katalysator Ru und/oder Ir enthält.

13. Verfahren nach einem der Ansprüche 1 bis 12, **dadurch gekennzeichnet, dass** der Katalysator mindestens einen Phosphordonorliganden enthält.

14. Verfahren nach einem der Ansprüche 1 bis 13, **dadurch gekennzeichnet, dass** die Umsetzung in Schritt (a) bei einer Temperatur von 20 bis 250 °C und bei Drücken von 0,1 bis 20 MPa absolut durchgeführt wird.

15. Verfahren nach einem der Ansprüche 1 bis 14, **dadurch gekennzeichnet, dass** die Umsetzung in Schritt (a) unter Zusatz von Basen erfolgt.

## Claims

1. A process for the preparation of primary amines by alcohol amination of alcohols with ammonia with the elimination of water, comprising the steps
(a) homogeneously catalyzed reaction of a reaction mixture comprising at least one alcohol, ammonia, at least one nonpolar solvent and at least one catalyst comprising at least one element selected from groups 8, 9 and 10 of the Periodic Table of the Elements in the liquid phase, giving a product mixture (P),
(b) phase separation of the product mixture (P) obtained in step (a), optionally after lowering the temperature, lowering the pressure and/or adding at least one polar solvent which has a miscibility gap with the nonpolar solvent, to give at least one polar product phase (A) and at least one nonpolar phase (B) comprising at least some of the catalyst used, and separating off the nonpolar phase (B),
(c) returning at least some of the nonpolar phase (B) to the reaction in step (a) and
(d) separating off the amination product from the polar product phase (A),
where the nonpolar solvent used in (a) and the catalyst used in step (a) are selected such that the catalyst accumulates in the nonpolar phase (B), where the alcohols have at least one primary or secondary alcohol group and at least a second functional group selected from primary or secondary alcohol group and primary amino group.

2. The process according to claim 1, wherein the primary amines are alkanolamines, diamines, triamines or polyamines.

3. The process according to claim 1 or 2, wherein the alcohols have at least one primary or secondary alcohol group.

4. The process according to any one of claims 1 to 3, wherein, in step (a) and/or (b), at least one polar solvent is used which has a miscibility gap with the nonpolar solvent and in which the amination product dissolves better than in the nonpolar solvent.

5. The process according to any one of claims 1 to 4, wherein the quantitative partition coefficient P_{MC} = [amount of dissolved catalyst in the nonpolar phase (B)]/[amount of dissolved catalyst in the polar product phase (A)] is at least 1.5.

6. The process according to any one of claims 1 to 5, wherein the quantitative partition coefficient P_{MA} = [amount of amination product in the polar phase (A)]/[amount of amination product in the nonpolar phase (B)] is at least 1.5.

7. The process according to any one of claims 1 to 6, wherein the at least one polar solvent is selected from water, dimethylformamide, formamide, acetonitrile, the alcohol used in step (a) and mixtures thereof.

8. The process according to any one of claims 1 to 7, wherein the nonpolar solvent is selected from saturated hydrocarbons such as hexane, heptane, octane and cyclohexane; linear and cyclic ethers such as diethyl ether, 1,4-dioxane, tert-butyl methyl ether, diglyme and 1,2-dimethoxyethane and aromatic hydrocarbons such as benzene, toluene, o-, m-, p-xylene and mesitylene, and mixtures thereof.

9. The process according to any one of claims 1 to 8, wherein, prior to separating off the amination product in step (d), the catalyst present in the polar product phase (B) is extracted with the nonpolar solvent used in step (a).

10. The process according to claim 9, wherein the extracted catalyst is returned at least partially to the reaction.

11. The process according to any one of claims 1 to 10, wherein, in step (d), the amination product is separated off from the polar solvent by distillation.

12. The process according to any one of claims 1 to 11, wherein the catalyst comprises Ru and/or Ir.

13. The process according to any one of claims 1 to 12, wherein the catalyst comprises at least one phosphorus donor ligand.

14. The process according to any one of claims 1 to 13, wherein the reaction in step (a) is carried out at a temperature of from 20 to 250°C and at pressures of from 0.1 to 20 MPa absolute.

15. The process according to any one of claims 1 to 14, wherein the reaction in step (a) takes place with the addition of bases.

## Revendications

1. Procédé pour la production d'amines primaires par amination d'alcools par de l'ammoniac avec élimination d'eau, comprenant les étapes
(a) mise en réaction, catalysée par catalyse homogène, d'un mélange réactionnel contenant au moins un alcool, de l'ammoniac, au moins un solvant non polaire et au moins un catalyseur contenant au moins un élément choisi dans les groupes 8, 9 et 10 du système périodique, dans la phase liquide, avec obtention d'un mélange produit (P),
(b) séparation de phases du mélange produit (P) obtenu dans l'étape (a) éventuellement après abaissement de la température, abaissement de la pression et/ou addition d'au moins un solvant polaire qui présente une non-miscibilité au solvant non polaire, avec obtention d'au moins une phase polaire de produit (A) et d'au moins une phase non polaire (B) contenant au moins une partie du catalyseur utilisé et séparation de la phase non polaire (B),
(c) recyclage d'au moins une partie de la phase non polaire (B) dans la réaction dans l'étape (a) et
(d) séparation des produits d'amination d'avec la phase de produit polaire (A),
le solvant non polaire utilisé en (a) et le catalyseur utilisé dans l'étape (a) étant choisis de manière que le catalyseur s'accumule dans la phase non polaire (B), les alcools comportant au moins un groupe alcool primaire ou secondaire et au moins un deuxième groupe fonctionnel choisi parmi un groupe alcool primaire ou secondaire et un groupe amino primaire.

2. Procédé selon la revendication 1, **caractérisé en ce que** pour ce qui est des amines primaires il s'agit d'alcanolamines, de diamines, triamines ou polyamines.

3. Procédé selon la revendication 1 ou 2, **caractérisé en ce que** les alcools comportent au moins un groupe alcool primaire ou secondaire.

4. Procédé selon l'une quelconque des revendications 1 à 3, **caractérisé en ce que** dans l'étape (a) et/ou l'étape (b) on utilise au moins un solvant polaire qui présente une non-miscibilité au solvant non polaire et dans lequel le produit d'amination se dissout mieux que dans le solvant non polaire.

5. Procédé selon l'une quelconque des revendications 1 à 4, **caractérisé en ce que** le coefficient de partage des quantités V_{MK} = [quantité du catalyseur dissous dans la phase non polaire (B)] / [quantité du catalyseur dissous dans la phase polaire de produit (A)] est d'au moins 1,5.

6. Procédé selon l'une quelconque des revendications 1 à 5, **caractérisé en ce que** le coefficient de partage des quantités V_{MA} = [quantité du produit d'amination dans la phase polaire (A)] / [quantité du produit d'amination dans la phase non polaire (B)] est d'au moins 1,5.

7. Procédé selon l'une quelconque des revendications 1 à 6, **caractérisé en ce que** ledit au moins un solvant polaire est choisi parmi l'eau, le diméthylformamide, le formamide, l'acétonitrile, l'alcool utilisé dans l'étape (a) et des mélanges de ceux-ci.

8. Procédé selon l'une quelconque des revendications 1 à 7, **caractérisé en ce que** le solvant non polaire est choisi parmi des hydrocarbures saturés tels que l'hexane, l'heptane, l'octane et le cyclohexane ; des éthers linéaires et cycliques tels que l'oxyde d'éthyle, le 1,4-dioxane, l'oxyde de méthyle et de tert-butyle, le diglyme et le 1,2-diméthoxyéthane et des hydrocarbures aromatiques tels que le benzène, le toluène, l'o-, le m-, le p-xylène et le mésitylène ainsi que des mélanges de ceux-ci.

9. Procédé selon l'une quelconque des revendications 1 à 8, **caractérisé en ce qu'**avant la séparation du produit d'amination dans l'étape (d) on extrait le catalyseur contenu dans la phase polaire de produit (B) à l'aide du solvant non polaire utilisé dans l'étape (a).

10. Procédé selon la revendication 9, **caractérisé en ce que** le catalyseur extrait est au moins en partie recyclé dans la réaction.

11. Procédé selon l'une quelconque des revendications 1 à 10, **caractérisé en ce que** dans l'étape (d) on sépare par distillation le produit d'amination d'avec le solvant polaire.

12. Procédé selon l'une quelconque des revendications 1 à 11, **caractérisé en ce que** le catalyseur contient du Ru et/ou de l'Ir.

13. Procédé selon l'une quelconque des revendications 1 à 12, **caractérisé en ce que** le catalyseur contient au moins un ligand donneur de phosphore.

14. Procédé selon l'une quelconque des revendications 1 à 13, **caractérisé en ce qu'**on effectue la réaction dans l'étape (a) à une température de 20 à 250 °C et sous des pressions absolues de 0,1 à 20 MPa.

15. Procédé selon l'une quelconque des revendications 1 à 14, **caractérisé en ce que** la réaction dans l'étape (a) s'effectue avec addition de bases.
